(19) Europäisches Patentamt
European Patent Office
Office européen des brevets

(11) Publication number: 0 352 228 A2

(12) # EUROPEAN PATENT APPLICATION

(21) Application number: 89810522.6

(22) Date of filing: 11.07.89

(51) Int. Cl.[5]: C 07 K 7/10
C 12 N 15/15, A 61 K 37/02
// A61K35/62

(30) Priority: 19.07.88 GB 8817160

(43) Date of publication of application:
24.01.90 Bulletin 90/04

(84) Designated Contracting States:
AT BE CH DE ES FR GB GR IT LI LU NL SE

(71) Applicant: CIBA-GEIGY AG
Klybeckstrasse 141
CH-4002 Basel (CH)

UCP GEN-PHARMA AG
Solothurnstrasse 24
CH-3422 Kirchberg (CH)

(72) Inventor: Wallace, Andrew, Dr.
Klybeckstrasse 122
CH-4057 Basle (CH)

Dennis, Stanley, Dr.
Maulbeerstrasse 37
CH-4058 Basle (CH)

Hofsteenge, Jan, Dr.
Mischelistrasse 15
CH-4153 Reinach (CH)

Stone, Stuart, R., Dr.
Claragraben 116
CH-4057 Basle (CH)

The applicant has filed a statement in accordance with Rule 28 (4) EPC (issue of a sample only to an expert). Accession number(s) of the deposit(s): DSM 3665 and DSM 4084.
Claims for the following Contracting States: ES + GR.

(54) **Novel proteins.**

(57) Novel polypeptides are provided which differ from desulphatohirudin by simple or multiple mutations. The novel polypeptides have valuable pharmacological properties and can be prepared by recombinant DNA techniques.

Fig. 2: Plasmid map of plasmid pJDB207/GAPFL-HIR

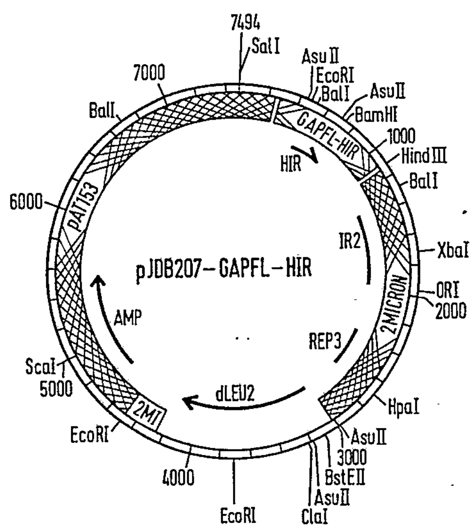


EP 0 352 228 A2

## Description

### Novel proteins

The present invention relates to novel proteins, to methods for the preparation thereof, to pharmaceutical compositions containing such proteins and to their use for the inhibition of enzymes.

Hirudin, the anticoagulant principle that occurs naturally in the leech Hirudo medicinalis, is a polypeptide of 65 amino acids with three disulphide bridges. Long known since the first investigations on leech saliva in 1884 and the pioneering work of Markwardt [Naturwissenschaften 42, 537 (1955); Methods Enzymol. 19, 924 (1970)], it was not until recently that the hirudin structure was elucidated by Dodt et. al. [FEBS Lett. 165, 180 (1984)]. The most recent findings are that hirudin exists in several variants, described as hirudin variant 1 (HV1), hirudin variant 2 (HV2), hirudin PA and other analogs.

Hirudin is the strongest thrombin inhibitor known, while other enzymes of the coagulation cascade are not affected. In contrast to heparin which is the preferred anticoagulant in conventional anticoagulation therapy, hirudin exerts its action directly on thrombin and, unlike the former, does not act through antithrombin III. The intersection between hirudin and thrombin occurs with extremely high potency. This potency is many orders of magnitude greater than the binding of thrombin to fibrinogen, to platelets of antithrombin III. Therefore, even low concentrations of hirudin are able to overcome all the interactions of thrombin and their biological consequences. Furthermore, hirudin has a low toxicity, is non antigenic and shows almost complete clearance via the kidneys in a biologically active form.

Recently, cDNAs and synthetic genes coding for hirudin or hirudin variants have been cloned and expressed in microbial hosts such as Escherichia coli and Saccharomyces cerevisiae (cf. European Patent Applications No. 158564 and 168342). Although the expression products lack the sulphate monoester group at $Tyr^{63}$- and were therefore desig nated "desulphatohirudins" - they turned out to exhibit approximately the same biological acitivity as natural hirudin in which the tyrosine residue is present as sulphate monoester.

Hirudin has been administered to man by both intravenous and subcutaneous routes. By intravenous route the mean elimination half life was found to be 0.84 h and approximately 50 % of the administered dose was excreted intact in the urine. After subcutaneous administration inhibitory levels of hirudin were found in the plasma for at least 4 h. Hirudin is therefore considered to be fairly short acting with a duration of action similar to heparin. Considering this drawback there is a strong need for selective antithrombotically active polypeptides exhibiting a longer half life in vivo. Corresponding polypeptides allowing once daily treatment will have advantages over existing therapies in deep vein and arterial thrombosis. It is an object of the present invention to provide such antithrombotically active polypeptides.

There is also a strong need for hirudin compounds which exhibit reduced antithrombotic potency. Reduction of the antithrombotic potency results in a reduction of the ability of these hirudin compounds to prevent thrombin interacting with its highest affinity ligands, notably the platelet receptor. However, by careful selection of the antithrombotic potency of the hirudin it retains its ability to inhibit fibrinogen clotting, a low affinity property of thrombin. Such hirudin compounds have the advantage in that they have inhibitory effects on thrombosis, a fibrin dominated event, but have little effect on haemostasis, platelet dominated effect. It is a further object of the present invention to provide such hirudin compounds with reduced antithrombotic potency.

Surprisingly, it was found that the increase in hydrophobicity of hirudin caused by site specific mutations of the nucleotides encoding basic amino acids (Lys) in the core region and/or by other modifications in said region leads to a reduced antithrombotic potency and/or a longer half life in vivo.

Accordingly, the presnt invention provides novel mutants of desulphatohirudin having the amino acid sequence

```
                                      10
H-Z -Val-Tyr-Thr-Asp-Cys-Thr-Glu-Ser-Gly-
   o
                                      20
 -Gln-Asn-Leu-Cys-Leu-Cys-Glu-Gly-Ser-Asn-
                                      30
 -Val-Cys-Gly-Gln-Gly-Asn-Z1-Cys-Ile-Leu-
                                      40
 -Gly-Ser-Asp-Gly-Glu-Z2-Asn-Gln-Cys-Val-
                                    50
 -Thr-Gly-Glu-Gly-Thr-Pro-Z3-Z4-Z5-Ser-
                                    60
 -Z6-Z7-Asp-Gly-Asp-Phe-Glu-Glu-Ile-Pro-

 -Glu-Glu-Tyr-Leu-Gln-OH
                              (I)
```

wherein $Z_o$ represents Val or the dipeptidyl radicals Gly-Val or Met-Val, $Z_1$ is Lys, Gln, Asn, Leu, Arg or Val, $Z_2$ represents Lys, Arg, Asn, Val, Leu or Gln, $Z_3$ represents Lys, Arg, Asn, Val or Leu, $Z_4$ represents Pro or Gly, $Z_5$ and $Z_7$ independently from each other represent Gln, Asn or Met, and $Z_6$ represents His, Gln or Asn, with the exception of compounds of the formula I in which $Z_o$ represents Val, $Z_1$ represents Lys or Gln, $Z_2$ represents Lys or Gln, $Z_3$ represents Lys, $Z_4$ represents Pro, $Z_5$ represents Gln, $Z_6$ represents Gln or His and $Z_7$ represents Asn, and salts thereof.

The mutants according to the invention differ from native desulphatohirudin ($Z_o$ is Val, $Z_1$, $Z_2$ and $Z_3$ are each Lys, $Z_4$ is Pro, $Z_5$ is Gln, $Z_6$ is His and $Z_7$ is Asn) with respect to 1 to 5, in particular 1 to 4, amino acid radicals.

In particular, the invention relates to mutants of desulphatohirudin of the formula I wherein $Z_o$ represents Val or the dipeptidyl radicals Gly-Val or Met-Val, $Z_1$ represents Lys, Gln, Asn or Arg, $Z_2$ represents Lys, Arg, Asn, Val or Gln, $Z_3$ represents Lys or Arg, $Z_4$ represents Pro or Gly, $Z_5$ represents Gln or Met, $Z_6$ represents His or Asn and $Z_7$ represents Asn or Met, with the exception of compounds of the formula I in which $Z_o$ represents Val, $Z_1$ represents Lys or Gln, $Z_2$ represents Lys or Gln, $Z_3$ represents Lys, $Z_4$ represents Pro, $Z_5$ represents Gln, $Z_6$ represents His and $Z_7$ represents Asn, and salts thereof.

Example of such desulphatohirudin mutants are [$Asn^{27}$]-desulphatohirudin, [$Asn^{36}$]-desulphatohirudin, [$Val^{36}$]-desulphatohirudin, [$Gly^{48}$]-desulphatohirudin, [$Met^{49}$]-desulphatohirudin, [$Met^{52}$]-desulphatohirudin, [$Asn^{51}$]-desulphatohirudin, [$Gln^{27}$, $Arg^{47}$]-desulphatohirudin, [$Gln^{27}$, $Gln^{36}$], $Arg^{47}$]-desulphatohirudin, [$Arg^{36}$, $Arg^{47}$]-desulphatohiurudin, [$Arg^{27}$, $Arg^{47}$]-desulphatohirudin, Glycyl-[$Gln^{27}$, $Gln^{36}$, $Arg^{47}$]-desulphatohirudin and Methionyl-[$Gln^{27}$, $Arg^{47}$]-desulphatohirudin.

The compounds of the invention can be in the free form but also in the form of their salts. As they contain free amino groups in several amino acid residues, the compounds of the invention can be in the form of acid addition salts. Suitable acid addition salts are in particular pharmacologically acceptable salts with conventional therapeutically acceptable acids. Representative inorganic acids are hydrohalic acids (such as hydrochloric acid), and also sulfuric acid, phosphoric acid and pyrophosphoric acid. Representative organic acids are in particular arenesulfonic acids (such as benzenesulfonic or p-toluenesulfonic acid), or lower alkanesulfonic acids (such as methanesulfonic acid), as well as carboxylic acids such as acetic acid, lactic acid, palmitic acid, stearic acid, malic acid, tartaric acid, ascorbic acid and citric acid. As, however, the compounds according to the invention also contain free carboxyl groups in several amino acid residues, which carboxyl groups impart acidic character to the entire peptide, they can also be in the form of salts with inorganic or organic bases, e.g. sodium, potassium, calcium or magnesium salts, or also ammonium salts derived from ammonia or a pharmacologically acceptable organic nitrogen-containing base. However, as they contain at the same time free carboxyl groups and free amino groups, they can also be in the form of inner salts. Pharmacologically acceptable salts are preferred.

The method for the preparation of the desulphatohirudin mutants according to the invention comprises culturing a microbial host strain which has been transformed with a hybrid vector comprising an expression cassette consisting of a promoter operably linked to a first DNA sequence encoding a signal peptide linked in the proper reading frame to a second DNA sequence coding for the desulphatohirudin mutant, and a DNA sequence containing transcription termination signals, and isolating the desulphatohirudin, mutant, and, if desired, converting an obtained polypeptide having free carboxy and/or amino groups into a salt or converting a salt obtained into the free compound.

Suitable microbial host strains include, for example, strains of Bacillus subtilis, Escherichia coli and Saccharomyces cerevisiae.

The transformed microbial host strains are cultured in a liquid medium containing assimilatable sources of carbon, nitrogen and inorganic salts, applying methods known in the art.

Various carbon sources are usable. Example of preferred carbon sources are assimilable carbohydrates, such as glucose, maltose, mannitol, fructose or lactose, or an acetate such as sodium acetate, which can be used either alone or in suitable mixtures. Suitable nitrogen sources include, for example, amino acids, such as casamino acids, peptides and proteins and their degradation products, such as tryptone, peptone or meat extracts, furthermore yeast extract, malt extract, corn steep liquor, as well as ammonium salts, such as ammonium chloride, sulphate or nitrate which can be used either alone or in suitable mixtures. Inorganic salts which may be used include, for example, sulphates, chlorides, phosphates and carbonates of sodium, potassium, magnesium and calcium. Additionally, the nutrient medium may also contain growth promoting substances. Substances which promote growth include, for example, trace elements, such as iron, zinc, manganese and the like, or individual amino acids.

Microbial host cells transformed with hybrid vectors tend to lose the latter. Such cells have to be grown under selective conditions, i.e. conditions which require the expression of a hybrid vector-encoded gene for growth. Most selective markers currently in use and present in the hybrid vectors according to the invention (infra) are genes coding for enzymes of amino acid or purine biosynthesis. This makes it necessary to use synthetic minimal media deficient in the corresponding amino acid or purine base. However, genes conferring resistance to an appropriate biocide may be used as well [e.g. a gene conferring resistance to the amino-glycoside G418]. Host cells transformed with vectors containing antibiotic resistance genes are grown in complex media containing the corresponding antibiotic whereby faster growth rates and higher cell densities are reached.

Host cells containing hybrid vectors with a constitutive promoter express the desulphatohirudin mutant gene controlled by said promoter without induction. However, it the desulphatohirudin mutant gene is under the control of a regulated promoter the composition of the growth medium has to be adapted in order to obtain maximum levels of mRNA transcripts, i.e. when using the PHO5 promoter in yeast the growth medium must contain a low concentration of inorganic phosphate for derepression of this promoter.

The cultivation is carried out by employing conventional techniques. The culturing conditions, such as temperature, pH of the medium and fermentation time are selected in such a way that maximal levels of desulphatohirudin mutants are produced. A chosen yeast or E. coli strain is preferably grown under aerobic conditions in submerged culture with shaking or stirring at a temperature of about 25° to 35° C, preferably at about 28° C, at a pH value of from 4 to 7, for example at approximately pH 5, and for at least 12 hours to 3 days, preferably for such a period that satisfactory yields of desulphatohirudin mutants are obtained.

Irrespective of the host strain, promoter and signal peptide used, most of the produced desulphatohirudin mutant is secreted into the culture medium or the periplasmic space whereas only a minor part remains associated with the cell interior. The precise ratio (secreted compounds/cell associated compounds) depends on the fermentation conditions.

The desulphatohirudin mutants can be isolated by conventional means. For example, the first step consists usually in separating the cells from the culture fluid by means of centrifugation. The resulting supernatant can be enriched for secreted desulphatohirudin mutants by treatment with polyethyleneimine so as to remove most of the non-proteinaceous material, and precipitation of the proteins by saturating the solution with ammonium sulphate. Host proteins, if present, can also be precipitated by means of acidification with acetic acid (for example 0.1 %, pH 4-5). A further enrichment of the desulphatohirudin mutant can be achieved by extracting the acetic acid supernatant with n-butanol. Other purification steps include, for example, desalination, chromatographic processes, such as ion exchange chromatography, gel filtration chromatography, partition chromatography, HPLC, reversed phase HPLC and the like. The separation of the constituents of the protein mixture is also effected by dialysis, according to charge by means of gel electrophoresis or carrier-free electrophoresis, according to molecular size by means of a suitable Sephadex column, by affinity chromatography, for example with antibodies, especially monoclonal antibodies, or with thrombin coupled to a suitable carrier for affinity chromatography, or by other processes, especially those known from the literature.

If it is desired to isolate desulphatohirudin mutants which have accumulated in the periplasmic space, some supplementary purification steps are required: The desulphatohirudin mutants are recovered by enzymatic removal of the cell wall (infra) or by treatment with chemical agents, e.g. thiol reagents or EDTA, or by subjecting the cell wall to osmotic shocks, which give rise to cell wall damages permitting the product to be released.

The test with anti-hirudin or anti-desulphatohirudin antibodies (for example, monoclonal antibodies obtainable from hybridoma cells), the thrombin test [M.U. Bergmeyer (ed.), Methods in Enzymatic Analysis, Vol. II, p. 314-316, Verlag Chemie, Weinheim (FRG) 1983] or the blood coagulation test [F. Markwardt et al., Thromb. Haemost. 47, 226 (1982)] can be used to detect the desulphatohirudin mutant activity in fractions obtained in the course of the purification procedure.

Depending on the method employed, the compounds of the invention are obtained in the free form or in the form of acid addition salts, inner salts or salts with bases. The free compound can be obtained in known manner from the acid addition salts or salts with bases. In turn, therapeutically acceptable acid addition salts can be obtained from the free compounds by reaction with acids, e.g. with those acids which form the above-mentioned salts, and by evaporation or lyophilisation. The inner salts can be obtained by adjusting the pH to a suitable neutral point.

The transformed microbial host cells according to the invention can be prepared by recombinant DNA techniques comprising the steps of
- preparing a hybrid vector comprising an expression cassette consisting of a promoter operably linked to a first DNA sequence encoding a signal peptide linked in the proper reading frame to a second DNA sequence coding for the desulphatohirudin mutant, and a DNA sequence containing transcription termination signals,
- transforming a microbial host strain with said hybrid vector,
- and selecting transformed microbial host cells from untransformed host cells.

Expression vectors

The hybrid vectors according to the invention comprise an expression cassette consisting of a promoter operably linked to a first DNA sequence encoding a signal peptide linked in the proper reading frame to a second DNA sequence coding for the desulphatohirudin mutant, and a DNA sequence containing transcription termination signals.

The selection of a suitable vector is determined by the microbial host cell provided for the transformation. Suitable hosts are those specified above, especially strains of Saccharomyces cerevisiae, and bacterial strains, especially strains of Escherichia coli and Bacillus subtilis.

Examples of vectors that are suitable for the expression of the desulphatohirudin mutant gene in an E. coli strain are bacteriophages, for example derivatives of the bacteriophage λ, or plasmids, such as the plasmid co1E1 and its derivatives, for example pMB9, pSF2124, pBR317 or pBR322. Suitable vectors contain a complete replicon and a marker gene, which renders possible the selection and identification of the microorganisms transformed by the expression plasmids by means of a phenotype feature. Suitable marker genes impart to the microorganism, for example, resistance to heavy metals, antibiotics such as ampicillin or tetracyclin, and the like.

Several promoters can be used for regulating the expression cassette in E. coli. Especially promoters of strongly expressed genes are used. Suitable promoters are the lac, tac, trp and lpp promoters, furthermore the phage λN or the phage λpL promoter, and others. In the present invention, the preferred promoter for use in E. coli is the lpp and the lac promoter.

Vectors suitable for replication and expression in S. cerevisiae contain a yeast-replication origin and a selective genetic marker for yeast. Hybrid vectors that contain a yeast replication origin, for example the chromosomal autonomously replicating segment (ars), are retained extrachromosomally within the yeast cell after transformation and are replicated autonomously during mitosis. Also, hybrid vectors that contain sequences homologous to the yeast 2μ plasmid DNA can be used. Such hybrid vectors are integrated by recombination in 2μ plasmids already present within the cell, or replicate autonomously. Suitable marker genes for yeast are especially those that impart antibiotic resistance to the host or, in the case of auxotrophic yeast mutants, genes that complement the host lesions. Corresponding genes impart, for example, resistance to the antibiotic cycloheximide or provide for prototrophy in an auxotrophic yeast mutant, for example the URA3, LEU2, HIS3 or the TRPI gene.

Preferably, yeast hybrid vectors furthermore contain a replication origin and a marker gene for a bacterial host, especially E. coli, so that the construction and the cloning of the hybrid vectors and their precursors can be carried out in E. coli. Promoters suitable for expression in yeast are, for example, those of the ADHI, ADHII, or PH05 gene, and also promoters involved in glycolysis, for example the PGK or the GAP promoter.

The DNA sequence encoding a signal peptide ("signal sequence") is derived from a gene of the microbial host coding for a polypeptide which is ordinarily secreted. When E. coli is used as the host microorganism the ompA, lpp, maltose binding protein, λ receptor, leucine binding protein or β-lactamase signal sequence may be chosen. For use in yeast the hirudin signal sequence obtainable from leech genome DNA can be chosen. More preferred signal sequences for use in yeast are, for example, the signal and prepro sequences of the yeast invertase, α-factor, pheromone peptidase (KEX1), "killer toxin" and repressible acid phosphatase (PH05) genes and the glucoamylase signal sequence from Aspergillus awamori. Alternatively, fused signal sequences may be constructed by ligating part of the signal sequence (if present) of the gene naturally linked to the promoter used (for example PH05), with part of the hirudin signal sequence. Those combinations are favoured which allow a precise cleavage between the signal sequence and the desulphatohirudin mutant amino acid sequence. Additional sequences, such as pro- or spacer-sequences which may or may not carry specific processing signals can also be included in the constructions to facilitate accurate processing of precursor molecules.

The DNA sequence containing transcription termination signals is preferably the 3′ flanking sequence of a gene derived from the selected microbial host which contains proper signals for transcription termination. Suitable 3′ flanking sequences are, for example, those of the gene naturally linked to the promoter used.

The hybrid vectors according to the invention can be prepared by methods known in the art, for example by linking the expression cassette consisting of a promoter operably linked to a first DNA sequence encoding a signal peptide linked in the proper reading frame to a second DNA sequence coding for the desulphatohirudin mutant, and a DNA sequence containing transcription termination signals, or the constituents of the expression cassette to the DNA fragments containing selective genetic markers and origins of replication for the selected microbial host in the predetermined order.

The DNA coding for the desulphatohirudin mutants according to the invention can be manufactured by methods known in the art. The methods for the manufacture of the DNA include excising a portion of the DNA comprising the codons for the undesired amino acid residues from the parental desulphatohirudin gene and replacing it with a DNA segment wherein said codons have been substituted with deoxyribonucleotide triplets coding for the desired amino acid residues, or accomplishing the mutation by means of site-directed mutagenesis.

For the preparation of the DNA coding for the desulphatohirudin mutant, excision of a portion of the desulphatohiridun DNA may be affected by using restriction enzymes. A prerequisite of this method is the availability of appropriate restriction sites in the vicinity of the codons to be altered. For example, a small restriction fragment containing the codon for an undesired amino acid is removed by endonuclease cleavage. A corresponding double stranded DNA sequence is prepared, for example by means of chemical synthesis, in which triplets coding for the desired amino acid are used. The DNA fragment is ligated in the proper orientation to the remaining large fragment to yield a double stranded DNA sequence coding for the mutant. For convenience and in order to facilitate handling of the mutant gene the latter is advantageously contained in a greater DNA segment provided with appropriate linkers which allow insertion and cloning of the segment in a cloning vector.

In a preferred embodiment of the present invention the preparation of DNAs coding for the desulphatohirudin mutants is effected by site-directed mutagenesis. This method is an in vitro mutagenesis procedure by which a defined site within a region of cloned DNA can be altered [cf. the review articles of M.J. Zoller and M. Smith, Methods Enzymol. 100, 468 (1983); D. Botstein and D. Shortle, Science 229, 1193 (1985)]. Mutagenesis can either be effected on the complete desulphatohirudin gene or on functional parts thereof containing the codon(s) for the undesired amino acid(s). After mutagenesis, the mutated functional part is linked to the other parts of the desulphatohirudin gene to yield the complete desulphatohirudin mutant DNA.

The method of mutating the desulphatohirudin gene or functional part thereof is characterized in that the single-stranded gene or a single-stranded DNA comprising the desulphatohirudin gene or part thereof is hybridized to an oligodeoxyribonucleotide primer which is complementary to the region of the hybrid to be mutated except for mismatch(es) that direct(s) the mutation, the hybridized oligodeoxyribonucleotide is used as a primer to initiate the synthesis of the complementary DNA strand, the resulting (partially) double-stranded DNA is transformed into a recipient microorganism strain, the microorganism strain is cultivated and tranformants containing DNA with the modified (mutant) desulphatohirudin gene are selected.

Transformed microbial hosts

The invention concerns furthermore a microbial host strain which has been transformed with a hybrid vector comprising an expression cassette consisting of a yeast promoter operably linked to a first DNA sequence encoding a signal peptide linked in the proper reading frame to a second DNA sequence coding for the desulphatohirudin mutant, and a DNA sequence containing transcription termination signals, and to the method for the production thereof comprising transforming said microbial host strain with said hybrid vector.

The microbial host strain are those specified above. The transformation with the hybrid vectors according to the invention is carried out, for example, in the manner described in the literature for S. cerevisiae [A. Hinnen et al., Proc, Natl. Acad. Sci. USA 75, 1929 (1978)], B. subtilis [Anagnostopoulos et al., J. Bacteriol. 81, 741 (1961)] and E. coli [M. Mandel et al., J. Mol. Biol. 53, 159 (1970)]. The isolation of the transformed host cells is effected advantageously from a selective nutrient medium to which there has been added, for example, the biocide against which the marker gene contained in the expression plasmid imparts resistance. If, for example, the hybrid vectors contain the $amp^R$ gene, ampicillin is accordingly added to the nutrient medium. Cells that do not contain the hybrid vector are destroyed in such a medium.

Pharmaceutical compositions

The novel mutants of desulphatohirudin obtainable in accordance with the present invention have valuable pharmacological properties and can, like hirudin extracted from leeches, be used prophylactically or, especially, therapeutically.

The desulphatohirudin mutants according to the invention are, like natural hirudin, potent inhibitors of thrombin. They have, for example, $K_i$ (desulphatohirudin mutant-thrombin complex dissociation constant) values of $10^{-9}$ to $10^{-12}$ M. The desulphatohirudin mutants are completely specific to thrombin and exhibit no interactions with other proteinases of the blood coagulation system. The toxicity is extremely low. Similarly, no hypersensitivity reactions or allergic reactions are observed. Moreover, the mutants of the invention have an in vivo duration of action comparable with or better than that of native desulphatohirudin.

The novel desulphatohirudin mutants according to the invention can therefore be used analogously to natural hirudin for the therapy and prophylaxis of thromboses and thromboembolisms, including the prophylaxis of post-operative thromboses, for acute shock therapy (for example for septic or polytraumatic shock), for the therapy of consumption coagulopathies, in haemodialyses, haemoseparations and in extracorporeal blood circulation.

The invention relates also to pharmaceutical compositions that contain at least one of the compounds according to the invention or a pharmaceutically acceptable salt thereof, optionally together with a pharmaceutically acceptable carrier and/or adjuncts.

These compositions can be used especially in the above-mentioned indications, when they are administered, for example, parenterally, such as intravenously, intracutaneously, subcutaneously or intramuscularly, or topically.

The invention relates also to the use of the novel compounds according to the invention and to pharmaceutical compositions containing them for the prophylactic and therapeutic treatment of the human or animal body, especially for the above-mentioned clinical syndromes, especially for inhibiting the coagulation of blood inside and outside the human or animal body.

The dosage depends especially on the specific form of administration and on the purpose of the therapy or prophylaxis. The size of the individual doses and the administration regime can best be determined by way of an individual judgement of the particular case of illness; the methods of determining relevant blood factors required for this purpose are familiar to the person skilled in the art. Normally, in the case of an injection the therapeutically effective amount of the compounds according to the invention is in a dosage range of from approximately 0.005 to approximately 01. mg/kg body weight. A range of from approximately 0.01 to approximately 0.05 mg/kg body weight is preferred. The administration is effected by intravenous, intramuscular or subcutaneous injection. Accordingly, pharmaceutical compositions for parenteral administration in single dose form contain per dose, depending on the mode of administration, from approximately 0.4 to approximately 7.5 mg of the compound according to the invention. In addition to the active ingredient these pharmaceutical compositions usually also contain a buffer, for example a phosphate buffer, which is intended to keep the pH value between approximately 3.5 and 7, and also sodium chloride, mannitol or sorbitol for adjusting the isotonicity. They may be in freeze-dried or dissolved form, it being possible for solutions advantageously to contain an antibacterially active preservative, for example from 0.2 to 0.3 % 4-hydroxybenzoic acid methyl ester or ethyl ester.

A composition for topical application can be in the form of an aqueous solution, lotion or gel, an oily solution or suspension or a fat-containing or, especially, emulsified ointment. A composition in the form of an aqueous solution is obtained, for example, by dissolving the active ingredients according to the invention, or a therapeutically acceptable salt thereof, in an aqueous buffer solution of from pH 4 to pH 6.5 and, if desired, adding a further active ingredient, for example an anti-inflammatory agent, and/or a polymeric binder, for example polyvinylpyrrolidone, and/or a preservative. The concentration of the active ingredient is from approximately 0.1 to approximately 1.5 mg, preferably from 0.25 to 1.0 mg, in 10 ml of a solution or 10 g of a gel.

An oily form of administration for topical application is obtained, for example, by suspending the active ingredients according to the invention, or a therapeutically acceptable salt thereof, in an oil, optionally with the addition of swelling agents, such as aluminium stearate, and/or surfactants (tensides) having HLB value ("hydrophilic-lipophilic balance") of below 10, such as fatty acid monoesters of polyhydric alcohols, for example glycerine monostearate, sorbitan monolaurate, sorbitan monostearate or sorbitan monooleate. A fat-containing ointment is obtained, for example, by suspending an active ingredient according to the invention, or a salt thereof, in a spreadable fatty base, optionally with the addition of a tenside having an HLB value of below 10. An emulsified ointment is obtained by triturating an aqueous solution of the active ingredient according to the invention, or a salt thereof, in a soft, spreadable base with the addition of a tenside having an HLB value of below 10. All these forms for topical application can also contain preservatives. The concentration of active ingredient is from approximately 0.1 to approximately 1.5 mg, preferably from 0.25 to 1.0 mg, in approximately 10 g of base.

In addition to the compositions described above that are intended for direct medicinal use in the body of a human or an animal, the present invention relates also to pharmaceutical compositions for medicinal use outside the living body of humans or animals. Such compositions are used especially as anticoagulant additives to blood that is being subjected to circulation or treatment outside the body (for example extracorporeal circulation of dialysis in artificial kidneys), preservation or modification (for example haemoseparation). Such compositions, such as stock solutions or alternatively compositions in single dose form, are similar in composition to the injection compositions described above; however, the amount or concentration of active ingredient is advantageously based on the volume of blood to be treated or, more precisely, on its thrombin content. In this connection it must be borne in mind that the active ingredients according to the invention (in free form) completely deactivate approximately 5 times the amount by weight of thrombin, are physiologically harmless even in relatively large amounts, and are eliminated from the circulating blood rapidly even in high concentrations so that there is not risk of overdose, even, for example, during transfusions. Depending on the specific purpose, the suitable dose is from approximately 0.01 to approximately 1.0 mg of the active ingredient/litre of blood, although the upper limit may still be exceeded without risk.

The invention relates especially to the desulphatohirudin mutants, hybrid vectors, microbial host strains transformed with such hybrid vectors, and to the processes for their manufacture as described in the Examples.

Brief description of the drawings

In the following experimental section various embodiments of the present invention are described with reference to the accompanying drawings in which:

Fig. 1 schematically illustrated the construction of plasmid pML350.

Fig. 2 schematically shows the plasmid map of plasmid pJDB207/GAPFL-HIR.

Experimental section

Example 1: Construction of the plasmid pML350 (see Fig. 1)

a) Digestion of the DNA of plasmid pIN-III-ompA-2

10 µg of plasmid pIN-III-ompA-2 [J. Ghrayeb et al., EMBO-J. 3, 2437 (1984)] are dissolved in 25 µl of 100 mM Tris-HCl pH 7.5, 50 mM NaCl and 100 µg/ml gelatine and are digested with the restriction endonucleases EcoRI and BamHI. The solution is adjusted to TNE and extracted with phenol/chloroform. The DNA is precipitated with ethanol. The vector DNA pIN-III-ompA-2/EcoRI/BamHI is isolated after electrophoresis in agarose by gel elution.

b) Digestion of the DNA of plasmid pML310

20 µg of the plasmid pML310 (see European Patent Application No. 168342) are digested in 50 µl of 100 mM Tris-HCl pH 7.5, 50 mM NaCl and 100 µg/ml gelatine with the restriction endonucleases EcoRI and BamHI. The solution is adjusted to TNE and extracted with phenol/chloroform. The DNA is precipitated with ethanol. The $F_1$-$F_2$-DNA (hirudin gene) is isolated after gel electrophoresis in agarose by gel elution.

c) Ligation of the $F_1$-$F_2$-DNA (hirudin gene) from pML310 with the vector DNA pIN-III-ompA-2/EcoRI/BamHI

1 µg of $F_1$-$F_2$-DNA (hirudin gene)/EcoRI/BamHI and 30 µg of the vector DNA pIN-III-ompA-2/EcoRI/BamHI are dissolved in 50 µl of 100 mM Tris-HCl pH 7.5, 50 mM NaCl and 100 µg/ml gelatine, adjusted to TNE. The solution is extracted with phenol/chloroform and the DNA is precipitated with ethanol. The DNA precipitate is dissolved in 20 µl of a solution of 50 mM Tris-HCl (pH 7.8), 10 mM $MgCl_2$, 10 mM DTT, 0.5 mM ATP, and 100 µg/l gelatine and treated with 25 units/µl $T_4$ DNA ligase (Biolabs) at 15°C for 3 h. By this way the recombinant plasmid pML350 is created, which contains the $F_1$-$F_2$-DNA (hirudin gene) inserted.

d) Transformation of E. coli HB101 with plasmid pML350

The E. coli HB101 cells pretreated with calcium that are prepared as described by Mandel et al. [J. Mol. Biol. 53, 159 (1970)].

The solution obtained in c), which contains the recombinant plasmid pML350, is heated at 65°C for 10 min in order to inactivate the $T_4$ DNA ligase and is then cooled to 37°C. 10 µl of the resulting reaction mixture was added to 150 µl of calcium-treated E. coli HB101 cells in 10 mM $MgCl_2$ and 10 mM Tris•HCl (pH 7.5) in a total volume of 200 µl.

Subsequently, this mixture is cooled in ice for 30 min, heated for 2 min at 42°C and then left to stand for 50 min in 1 ml of L-medium (Bacto tryptone 10 g/l; Bacto yeast extract 5 g/l; NaCl 5 g/1; glucose 5 g/l; ampicillin 0.1 g/l) at 37°C. The mixture is then spread out in aliquots of 0.2 ml on 5 agar plates (McConkey Agar, Difco), which contain 60 µg/ml of ampicillin (Serva). The agar plates are then maintained at 37°C for 16-18 hours. 185 ampicillin resistant colonies of transformed E. coli HB101 cells are obtained.

e) Screening the colonies that contain $F_1$-$F_2$-DNA

6 transformed colonies (Example 1d) are pressed off onto nitrocellulose filter B85 (Schleicher and Schull). In accordance with Grunstein and Hogness [Proc. Natl. Acad. Sci. USA 72, 3961 (1979)] the colonies are lysed and their denatured DNA is fixed on the filter. Subsequently prehybridization of the filters is carried out in 20 ml (per filter) of 4xSET [= solution of 30 mM Tris•HCl (pH 8), 150 mM NaCl, 1mM EDTA], 0.1 % (w/v) Ficoll 400 (Pharmacia), 0.5 % SDS,d 50 µg/ml denatured calf thymus DNA for 4 h at 64°C. Subsequently the nitrocellulose filters are treated in 20 ml (per filter) of 5xSET (w/v), 0.1 % (w/v) Ficoll 400, 0.2 % SDS and 50 µl/ml denatured calf thymus DNA for 16 h at 64°C with the $^{32}P$ radioactively labelled probe (approximately $10^3$-$10^4$ Cerencov cpm per filter). A mixture consisting of oligonucleotides 46/64 complementary, 1/58, 96/67 and 154/64 complementary (see European Patent Application No. 168342) is used as probe.

Subsequently, the filters are washed twice in 2xSET, 0.2 % SDS at room temperature, then twice in 2xSET, 0.5 % SDS at 60°C (first for 30 min, then for 60 min). The filters are then dried between 3 MM paper (Whatman) and placed at -80°C on an X-ray film (Fuji) with an intensifying screen (Ilford) for 1-2 days.

The resulting autoradiogram shows 5 positive colonies (clones) which can be used for further processing, one of which received the designation pML350.

Example 2: Construction of plasmid pBH109

As plasmid pML350 originates from plasmid pIN-III-ompA-2 including a multi-cloning linker (EcoRI, HindIII, BamHI sites) it contains 12 additional base pairs in front of the mature hirudin gene coding for Ala, Gln, Phe, Met. To get mature desulphatohirudin expressed these 12 base pairs are looped out by in vitro mutagenesis making use of a 27mer oligonucleotide.

a) Preparation of pML350/XbaI/BAmHI (SI)

5 µg of plasmid pML350 are digested with the endonucleases XbaI and BamHI. The larger of the two XbaI-BamHI fragments (SI) is isolated by gel elution after electrophoresis on agarose and dissolved in 1 mM Tris-HCl pH 7.5, 0.1 mM EDTA.

b) Preparation of pML350/PvuI (SII)

5 µg of plasmid pML350 are digested with the endonuclease PvuI. The linearized DNA pML350/PvuI is subsequently digested with 3 units of intestinal alcaline phosphatase (Boehringer) for 30 min at 37°C. The enzyme is inactivated by heating the solution for 60 min at 65°C. The linear pML350/PvuI (SII) DNA is isolated by gel elution after electrophoresis on agarose and dissolved in 1 mM Tris-HCl pH 7.5, 0.1 mM EDTA.

c) Preparation of the oligonucleotide (27 mer) I27

In analogy to the procedure described in European Patent Application No. 168342 the following DNA fragment (designated I27) has been synthesized:

```
5'-GTA GCG CAG GCC GTT GTT TAC ACC GAC-3'
                  I27
```

The phosphorylation at the 5′ ends was done with [$\gamma$-$^{32}$P]-ATP and $T_4$ polynucleotide kinase (Boehringer) as described [Molecular Cloning, A Laboratory Manual (ed. T. Maniatis et al.), Cold Spring Harbor Lab. (1982), p. 125]

d) Construction of plasmid pBH109

0.3 µg each of SI DNA and of SII DNA are mixed with 40 pmol of the phosphorylated DNA fragment I27 in 27 µl of 1 mM Tris-HCl pH 7.5, 0.1 mM EDTA. To the mixture 3 µl of 10X polymerase-ligase buffer (1M NaCl, 65mM Tris-HCl pH 7.5, 80 mM $MgCl_2$ and 10 mM β-mercaptoethanol) are added. This mixture is heated for 3 min in a boiling water bath to denature the DNA fragments. Subsequently, the mixture is gradually cooled (about 1°C/min) to 30°C and incubated at this temperature for 30 min. Further the mixture is incubated at 4°C for 30 min and afterwards for 10 min in ice.

12 µl of the four deoxyribonucleotide phosphates (7.5 mM each), 6 µl of 10 mM ATP, 6 µl of $T_4$ DNA ligase (2.5 U/µl) and 1.2 µl of Klenow DNA polymerase (Boehringer, 5 U/µl) are added and the DNA mixture (total volume 55 µl) is incubated for 16 h at 12.5°C.

The DNA mixture is digested with 2 units of endonuclease EcoRI for 1 h at 37°C in order to destroy unchanged starting plasmid pML350. With this procedure, plasmid pBH109 is formed. Plasmid pHB109 contains the lpp promoter and the lac promoter/operator operably linked to the ompA-2 signal sequence linked in frame to the gene coding for mature desulphatohirudin.

e) Transformation of E. coli HB101 with plasmide pBH109

The transformation with calcium treated E. coli HB101 cells is done as described in Example 1d. The total reaction mixture used is 55 µl.

f) Screening of the colonies which contain plasmid pBH109

100 transformed colonies are cultivated, from each colony plasmid DNA is prepared and digested with EcoRI. All plasmid DNAs, which are not digestable with EcoRI are potent plasmids pBH109 which is lacking the EcoRI site.

Two positive identical colonies have been identified. One of them is selected and designated pBH109.

The correct sequence of the $F_1$-$F_2$-DNA following the ompA-2 leader sequence is confirmed by sequence analysis.

Example 3: Mutation of the residue Lys27 of hirudin to Asn27 using single stranded M13mp19/hirudin

```
                    24      26      28      30
coding strand      Gln Gly Asn Lys Cys Ile Leu
of hirudin       5'CAG GGT AAC AAA TGC ATC CTG 3'

mutagenic
primer 1         3'GTC CCA TTG TTA ACG TAG GAC 5'

mutated          5'CAG GGT AAC AAT TGC ATC CTG 3'
coding strand      Gln Gly Asn Asn Cys Ile Leu
```

Mutagenic primers are synthesised using the phosphoramidite method [M.H. Caruthers, in Chemical and Enzymatic Synthesis of Gene Fragments (H.G. Gassen and A. Lang, Eds.) Verlag Chemie, Weinheim, Federal Republic of Germany] on an Applied Biosystems (Model 380B) synthesiser.

I. Preparation of M13mp19/hirudin.

A. XbaI-BamHI cut M13mp19 DNA.

To 5 µl M13mp19 double stranded DNA (ds-DNA; 0.1 µg/ml; BRL) are added 2 µl React 2 (500 mM Tris-HCl, pH 8.0; 100 mM $MgCl_2$, 500 mM NaCl) (BRL), 1 µl XbaI (10 U/µl), 0.5 µl BamHI (10 U/µl) and 12 µl $H_2O$. After incubation at 37°C for 1.5 h, 0.5 µl BamHI, 2.5 µl React 3 (500 mM Tris-HCl, pH 8.0; 100 mM $MgCl_2$; 1000 mM NaCl) (BRL), and 2 µl $H_2O$ are added and incubation is continued at 37°C for 1 h. The volume is made up to 100 µl with $H_2O$. The ds-DNA is isolated by phenol extraction and ethanol precipitation, and dissolved in 30 µl of TE buffer (Tris-HCl 10 mM, EDTA 1 mM, pH 8.0).

B. Insert DNA.

Five µg of the plasmid pBH109 are cut with XbaI and BamHI as described above and the digest is electrophoresed for 3 h at 150 volt using a 3.5 % polyacrylamide gel with 1x TBE buffer (10x TBE buffer: 108 g Tris, 55 g boric acid, 9.3 g EDTA•$2H_2O$/1). The XbaI-BamHI fragment containing the hirudin gene (250 bp) is visualised under UV light after immersing the gel in 400 ml 1x TBE buffer containing 10 µl of ethidium bromide solution (10 µg/ml in water). The part of the gel containing the restriction fragment is cut from the gel and placed in a dialysis bag with 500 µl of 0.5x TBE, and the DNA is electroeluted in a BIO-RAD minigel electrophoresis apparatus using 0.5x TBE as the running buffer at 170 volt for 30 min. The DNA is loaded onto an Elutip-d column (Schleicher & Schull) equilibrated with 0.5x TBE. The column is washed with 2ml of 0.5x TBE and the DNA is eluted with 1 M NaCl in 0.5x TBE (1 ml). The DNA is precipitated with ethanol and redissolved in 10 µl of TE buffer.

C. Ligation of XbaI-BamHI hirudin insert into M13mp19 and preparation of single stranded DNA.

Five µl XbaI-BamHI hirudin insert, 2 µl XbaI-BamHI cut M13mp19, 1 µl 10x ligase buffer (50 mM Tris-HCl, pH 7.5, 10 mM $MgCl_2$, 10 mM dithiothreitol), 1 µl ATP, 1.5 µl T4 DNA ligase (BRL; 1 U/µl) are mixed and incubated overnight at 14°C. Five µl of ligation mixture are used to transform E. coli JM101 competent cells according to the method of J. Messing [Methods in Enzymology 101, 21-78 (1983)]. Twelve clear plaques are picked and single stranded DNA (ss-DNA) is prepared from each plaque as described by J. Messing (supra). The DNA designated M13mp19/hirudin is redissolved in 50 µl of TE buffer (0.1-0.5 µg/µl).

II. Site-directed mutagenesis.

A. Phosphorylation of mutagenic primer.

200 pmol (23 µl) of mutagenic primer 1 (see above) is phosphorylated by adding 3 µl 10x kinase buffer (1M Tris-HCl, 0.1M $MgCl_2$, 0.1M dithiothreitol, pH 8.3) 3 µl 10 mM ATP and 1 µl T4 polynucleotide kinase (BRL, 10 U/µl). After incubation at 37°C for 1 h, the reaction is stopped by heating at 65°C for 10 min.

B. Annealing of the mutagenic primer 1 to the single-stranded M13mp19/hirudin template.

Six µl (0.5 µg) of single-stranded M13mp19/hirudin (Section I-C) is incubated with 3 µl (20 pmol) of the phosphorylated mutagenic oligodeoxyribonucleotide (6.6 pmol/µl) and 1 µl buffer A (0.2M Tris-HCl, pH 7.5, 0.1M $MgCl_2$, 0.5M NaCl, 0.01M DTT) at 70°C for 5 min, and cooled slowly to room temperature over 30 min.

C. Extension-ligation reaction.

To the above annealed mix is added 1 µl buffer B (0.2M Tris-HCl, pH 7.5, 0.1M $MgCl_2$, 0.01M DTT), 1 µl 10mM ATP, 4µl 2mM dNTP mixture, 5 µl T4 DNA polymerase (Boehringer, 1 U/µl), 5µl T4 DNA ligase (BRL, 1 U/µl). This mixture is incubated at 16°C for 3 h. The reaction is stopped by incubating at 65°C for 10 min.

D. Transformation and preparation of single-stranded mutant DNA

The ligation mixture is diluted 1:20 with sterile $H_2O$, and 1 µl, 5 µl, as well as 1 µl undiluted ligation mixture is used to transform competent E. coli BMH 71-81 mut S cells [B. Kramer, W. Kramer and H.-J. Fritz, Cell 38, 879-887 (1984)]. The cells are plated out as described in the "M13 cloning and sequencing Handbook" (published by Amersham). Twelve colourless plaques are picked and ss-DNA is prepared as described in section I-C.

E. Screening of single-stranded DNA for mutant.

To screen for mutated single-stranded DNA, each of the 12 ss-DNA samples is sequenced by the dideoxynucleotide chain termination method [F. Sanger, S. Nickler and A.R. Coulson, Proc. Natl. Acad. Sci. USA 74, 5463-5467 (1977)]. Initially, only the dideoxynucleotide complementary to the expected mutated base is used in the reaction. Subsequently, the ss-DNA from several positive mutants are sequenced to establish the full DNA sequence of the mutant using T7 DNA polymerase (Sequenase, USB) following the method of Tabor and Richardson [Proc. Natl. Acad. Sci. USA 84, 4767-4771 (1987)]. The expected base change encoding Lys → Asn mutation at position 27 of the recombinant hirudin are observed in the DNA sequence. The phage DNA having the expected sequence is designated M13mp19/hirudin K27N.

F. Preparation of replicative form (RF) DNA from single-stranded M13mp19/hirudin K27N phage DNA.

Competent E. coli JM101 cells are transformed with 10-20 ng of single-stranded hirudin K27N mutant DNA and ds-DNA is prepared as described in the "M13 cloning and sequencing Handbook" (published by Amersham). A yield of 40-50 μg of ds-DNA is obtained from 100 ml of culture.

G. Isolation of mutant hirudin XbaI-BamHI insert.

The mutated hirudin XbaI-BamHI insert is cut out of 25 μg of the ds-DNA and purified as described in section IB. The DNA is dissolved in 20 μl of sterile water.

H. Preparation of XbaI-BamHI cut pIN-III-ompa-2 vector DNA

A digest of approximately 1.5 μg pIN-III-ompA-2 plasmid is made by adding 6 μl React 2 buffer (BRL), 2 μl (20 Units) XbaI, 1 μl BamHI (10 Units), 1 μl EcoRI (10 Units) and 37 μl $H_2O$ (total volume 50 μl), and incubation for 3 h at 37°C. 1 μl (10 Units) BamHI, 1 μl (10 Units) EcoRI, 5 μl React 3 (BRL) and 12 μl $H_2O$ are added and incubation continued for 1 h at 37°C.

I. Ligation of mutant hirudin K27N XbaI-BamHI insert DNA into XbaI-BamHI cut pIN-III-ompA-2 plasmid.

Nine μl hirudin K27N XbaI-BamHI insert DNA, 2 μl XbaI-BamHI cut pIN-III-ompA-2 vector DNA, 3 μl 10x ligation buffer (BRL) and 1 μl (1 U/μl) T4 DNA ligase (BRL) are mixed and incubated at 14°C for 16-20 h.

III. Expression of mutant [$Asn^{27}$]-desulphatohirudin in E. coli JM101.

A. Transfection into E. coli strain JM101.

Five μl of ligation mixture is used to transform 0.3 ml of E. coli JM101 competent cells according to the method of J. Messing (supra). Three ml of 2xYT/Ampicillin (50 μg ampicillin/ml 2xYT) is added to the sample and the cells allowed to grow at room temperature for 1 h. A 1 ml sample of the culture is then taken and poured onto an LB/Ampicillin (50 μg ampicillin/ml LB-agar) plate and grown overnight at 37°C. The transforming plasmid DNA is referred to as pIN -III-ompA-2/HIR-K27N.

B. Selection of [$Asn^{27}$]-desulphatohirudin expressing colonies.

Ten bacterial colonies from the LB/Ampicillin plates are picked and grown separately for 5 h at 37°C in 5ml LB/Ampicillin (50 μg Ampicillin/ml LB). 1 ml samples are taken from each culture tube and the cells recovered by centrifugation (3000xg for 5 min). Each sample of cells is osmotically shocked by treatment with 100 μl of 10 mM Tris-HCl, pH 8.1 for 30 min at 0°C to release the material in the periplasmic space of the bacteria. The cells are removed by centrifugation as before, and the supernatant is tested for hirudin activity as described in Section V-B. The sample which gives the highest inhibitory activity is selected for batch culture.

C. Batch culture and isolation of [$Asn^{27}$]-desulphatohirudin.

The remaining quantity (4 ml) of cells from the most active sample is used to inoculate 1 l of LB/Ampicillin (50 μg Ampicillin/ml LB). The culture is grown overnight at 37°C, and the cells are recovered by centrifugation (3000xg for 15 min.). The cell pellet is osmotically shocked by resuspension in 50 ml of 10 mM Tris-HCl, pH 8.1 at 0°C for 1 h. The cells are removed from the periplasmic fraction by centrifugation at 6000xg for 10 min.

D. Purification of [$Asn^{27}$]-desulphatohirudin.

The pH of the periplasmic fraction is adjusted to 6.5 with 0.1M HCl and filtered through a 0.45 μm filter (Nalgene). The protein is loaded onto a Mono-Q column FPLC system (Fast Protein Liquid Chromatography, Pharmacia-LKB) equilibrated with 50mM bis-Tris-HCl buffer pH 6.5. the desulphatohirudin mutant is eluted from the column using a linear salt gradient of 0-300 mM NaCl in bis-Tris-HCl pH 6.5 over 45 min 0.8 ml fractions of the column eluate are collected and tested for hirudin activity as described in Section III-B. The desulphatohirudin mutant-containing fractions are pooled, filtered as above and chromatographed on a Millipore-Waters HPLC system using a Brownlee Labs C8 reversed-phase HPLC column equilibrated with 0.09 % (v/v) trifluoroacetic acid in $H_2O$. The hirudin mutant is eluted from the column with a linear gradient of 7 to 28 % (v/v) acetonitrile in 0.09 % (v/v) trifluoroacetic acid in $H_2O$. [$Asn^{27}$]-desulphatohirudin having a purity of about or more than 98 % elutes as a single peak at 28 min.

IV. Protein chemical characterization

Purified [$Asn^{27}$]-desulphatohirudin has been characterized by determining its amino acid composition, N-terminal sequence, and by peptide maping.

Two to five μg protein are hydrolysed in glass tubes in the vapor of 6N HCl at 110°C [R.L. Henrikson, and S.C. Meredith, Anal. Biochem. 136, 65-74 (1984)]. The amino acids are dried under vacuum, derivatized with phenylisothiocyanate, and separated on a reversed phase column [B.A. Bidlingmeyer, S.A. Cohen and T.L. Taruin, J. Chromatography 336, 93-104 (1984)]. The amino acid composition is calculated using a molecular weight of 6889 D.

Approximately 50 pmol of [$Asn^{27}$]-desulphatohirudin are subjected to 5 cycles of automated Edman degradation, using an Applied Biosystems 470A gas phase sequencer [M.W. Hunkapiller and L.E. Hood, Methods in Enzymology 91, 486-494, (1983)], to establish the N-terminal amino acid sequence. the resulting phenylthiohydantoin amino acids are separated on a reversed phase HPLC system [R.M. Hewick, M.W. Hunkapiller, L.E. Hood and W.J. Dreyer, J. Biol. Chem. 256, 7990-7997 (1981)], using an Applied Biosystems type 120 on-line analyser.

To obtain a peptide map of [$Asn^{27}$]-desulphatohirudin, 50 µg of protein are dried under vacuum, and treated with performic acid for 1 h at 37° C [C.H.W. Hirs, Methods in Enzymology 11, 197-199 (1967)]. 45 µl of cold water are added to the reaction mixture, frozen and dried under vacuum (twice). The oxidized hirudin mutant is dissolved in 50 µl of 50 mM $NH_4HCO_3$ and digested with 1 µg thermolysin (Boehringer for 4 h at 37° C. Prior to fractionation, the digest is dried under vacuum, and dissolved in 0.1 % trifluoroacetic acid (v/v) in $H_2O$. Peptides are purified by reversed phase HPLC, using a Vydac C18 column, equilibrated in 0.1 % trifluoroacetic acid in $H_2O$. Peptides are eluted with a linear gradient of 0-28 % acetonitrile over 90 min, at a flow rate of 1 ml/min.

The amino acid sequence of the peptide containing the mutations is sequenced as described above to confirm the amino acid substitutions Lys27 → Asn27.

Example 4: Mutation of the residue Lys 36 of hirudin to Asn 36

```
                          34        36        38
coding strand      Asp Gly Glu Lys Asn Gln Cys
of hirudin       5'GAC GGT GAA AAA AAC CAG TGC 3'

mutagenic
primer 2         3'CTG CCA CTT TTA TTG GTC ACG 5'

mutated          5'GAC GGT GAA AAT AAC CAG TGC 3'
coding strand      Asp Gly Glu Asn Asn Gln Cys
```

Site-directed mutagenesis is performed using mutagenic primer 2 and M13mp19/hirudin exactly as described in Example 3 (Sections I and II). The transforming plasmid DNA is referred to as pIN-III-ompA-2/HIR-K36N and the mutant protein is expressed in E. coli and purified as described for Example 3 (Section III). The identity of the mutant protein in which Lys 27 is replaced by Asn 27 is confirmed by amino acid composition and N-terminal sequence analysis (Example 3; Section IV) and its inhibitory properties are determined (Example 19). The mutant is designated [$Asn^{36}$]-desulphatohirudin.

Example 5: Mutation of the residue Lys 36 of hirudin to Val 36

```
                           34        36        38
coding strand       Asp Gly Glu Lys Asn Gln Cys
of hirudin       5' GAC GGT GAA AAA AAC CAG TGC 3'

mutagenic
primer 3         3' CTG CCA CTT CAT TTG GTC ACG 5'

mutated          5' GAC GGT GAA GTA AAC CAG TGC 3'
coding strand       Asp Gly Glu Val Asn Gln Cys
```

The procedures given above for Examples 3 and 4 are repeated using mutagenic primer 3 to obtain and characterize the desired mutant protein in which Lys 36 is replaced by Val 36. The transforming plasmid DNA is referred to as pIN-III-ompA-2/HIR-K36V. The mutant is designated [Val$^{36}$]-desulphatohirudin.

Example 6: Mutation of the residue Pro 48 of hirudin to Gly 48

```
                     44      46      48      50
coding strand        Gly Thr Pro Lys Pro Gln Ser His
of hirudin        5' GGT ACC CCG AAA CCG CAG TCT CAC 3'

mutagenic
primer 4          3' CCA TGG GGC TTT CCC GAC AGA GTG 5'

mutated           5' GGT ACC CCG AAA GGG CAG TCT CAC 3'
coding strand        Gly Thr Pro Lys Gly Gln Ser His
```

The procedures given above for Examples 3 and 4 are repeated using mutagenic primer 4 to obtain and characterize the desired mutant protein in which Pro 48 is replaced by Gly 48. The transforming plasmid DNA is referred to as pIN-III-ompA-2/HIR-P48G. The mutant is designated [Gly$^{48}$]-desulphatohirudin.

Example 7: Mutation of the residue Gln 49 of hirudin to Met 49

```
                         47      49
coding strand        Pro Lys Pro Gln Ser His Asn
of hirudin        5' CCG AAA CCG CAG TCT CAC AAC 3'

mutagenic         3' GGC TTT GGC TAC AGA GTG TTG 5'
primer 5

mutated           5' CCG AAA CCG ATG TCT CAC AAC 3'
coding strand        Pro Lys Pro Met Ser His Asn
```

The procedures given above for Examples 3 and 4 are repeated using mutagenic primer 5 to obtain and characterize the desired mutant protein in which Gln 49 is replaced by Met 49. The transforming plasmid DNA is referred to as pIN-III-ompA-2/HIR-Q49M. The mutant is designated [Met$^{49}$]-desulphatohirudin.

Example 8: Mutation of the residue Asn 52 of hirudin to Met 52

```
                                 52
coding strand      Gln Ser His Asn Asp Gly Asp
of hirudin     5'  CAG TCT CAC AAC GAC GGT GAC 3'

mutagenic
primer 6       3'  GTC AGA GTG TAC CTG CCA CTG 5'

mutated        5'  CAG TCT CAC ATG GAC GGT GAC 3'
coding strand      Gln Ser His Met Asp Gly Asp
```

The procedures given above for Examples 3 and 4 are repeated using mutagenic primer 6 to obtain and characterize the desired mutant protein in which Asn 52 is replaced by Met 52. The transforming plasmid DNA is referred to as pIN-III-ompA-2/HIR-N52M. The mutant is designated [$Met^{52}$]-desulphatohirudin.

Example 9: Mutation of the residue His 51 of hirudin to Asn 51

```
                                 51
coding strand      Pro Gln Ser His Asn Asp Gly
of hirudin     5'  CCG CAG TCT CAC AAC GAC GGT 3'

mutagenic      3'  GGC GTC AGA TTG TTG CTG CCA 5'
primer 7

mutated        5'  CCG CAG TCT AAC AAC GAC GGT 3'
coding strand      Pro Gly Ser Asn Asn Asp Gly
```

The procedures given above for Examples 3 and 4 are repeated using mutagenic primer 7 to obtain and characterize the desired mutant protein in which His 51 is replaced by Asn 51. The transforming plasmid DNA is referred to as pIN-III-ompA-2/HIR-H51N. The mutant is designated [$Asn^{51}$]-desulphatohirudin.

Example 10: Mutation of the residue Lys 27 of hirudin to Gln 27, and of the residue Lys 47 to Arg 47

A: Mutation of Lys 27 to Gln 27

```
                                 27
coding strand      Gln Gly Asn Lys Cys Ile Leu
of hirudin     5'  CAG GGT AAC AAA TGC ATC CTG 3'

mutagenic      3'  GTC CCA TTG GTT ACG TAG GAC 5'
primer 8A

mutated        5'  CAG GGT AAC CAA TGC ATC CTG 3'
coding strand      Gln Gly Asn Gln Cys Ile Leu
```

B: Mutation of Lys 47 to Arg 47

```
                                47
coding strand      Gly Thr Pro Lys Pro Gln Ser
of hirudin      5' GGT ACC CCG AAA CCG CAG TCT 3'

mutagenic       3' CCA TGG GGC TCT GGC GTC AGA 5'
primer 8B

mutated         5' GGT ACC CCG AGA CCG CAG TCT 3'
coding strand      Gly Thr Pro Arg Pro Gln Ser
```

The procedures given above for Examples 3 and 4 are repeated using mutagenic primers 8A and B to obtain and characterize the desired mutant protein in which Lys 27 is replaced by Gln 27 and Lys 47 by Arg 47. The transforming plasmid DNA is referred to as pIN-III-ompA-2/HIR-K27Q,K47R. The mutant is designated [$Gln^{27}$,$Arg^{47}$]-desulphatohirudin.

Example 11: Mutation of the residue Lys 27 of hirudin to Gln 27, the residue Lys 36 to Gln 36, and of the residue Lys 47 to Arg 47.

A: Mutation of Lys 36 to Gln 36

```
                        34      36      38
coding strand      Asp Gly Glu Lys Asn Gln Cys
of hirudin      5' GAC GGT GAA AAA AAC CAG TGC 3'

mutagenic
primer 9        3' CTG CCA CTT GTT TTG GTC ACG 5'

mutated         5' GAC GGT GAA CAA AAC CAG TGC 3'
coding strand      Asp Gly Glu Gln Asn Gln Cys
```

B: Mutation of Lys 27 to Gln 27

The mutation of Lys 27 to Gln 27 is performed according to example 10 A.

C: Mutation of Lys 47 to Arg 47

The mutation of Lys 47 to Arg 47 is performed according to example 10B.

The procedure given above for Examples 3 and 4 are repeated using mutagenic primers 8A, 9 and 8B to obtain and characterize the desired mutant protein in which Lys 27 is replaced by Gln 27, Lys 36 is replaced by Gln 36 and Lys 47 is replaced by Arg 47. The transforming plasmid DNA is referred to as pIN-III-ompA-2/HIR-K27Q,K36Q,K47R. The mutant is designated [$Gln^{27}$,$Gln^{36}$,$Arg^{47}$]-desulphatohirudin.

Example 12: Mutation of the residue Lys 36 to Arg 36, and of the residue Lys 47 to Arg 47.

A: Mutation of Lys 36 to Arg 36

```
                         34      36      38
coding strand        Asp Gly Glu Lys Asn Gln Cys
of hirudin        5' GAC GGT GAA AAA AAC CAG TGC 3'

mutagenic
primer 10         3' CGT CCA CTT TCT TTG GTC ACG 5'

mutated           5' GAC GGT GAA AGA AAC CAG TGC 3'
coding strand        Asp Gly Glu Arg Asn Gln Cys
```

B: Mutation of Lys 47 to Arg 47

The mutation of Lys 47 to Arg 47 is performed according to example 10 B.

The procedures given above for Examples 3 and 4 are repeated using mutagenic primers 8B and 10 to obtain and characterize the desired mutant protein in which Lys 36 is replaced by Arg 36 and Lys 47 is replaced by Arg 47. The transforming plasmid DNA is referred to as pIN-III-ompA-2/HIR-K36R,K47R. The mutant is designated [$Arg^{36}$,$Arg^{47}$]-desulphatohirudin.

Example 13: Mutation of the residue Lys 27 to Arg 27, and of the residue Lys 47 to Arg 47.

A: Mutation of Lys 27 to Arg 27

```
                         25      27      29
coding strand        Gln Gly Asn Lys Cys Ile Leu
of hirudin        5' CAG GGT AAC AAA TGC ATC CTG 3'

mutagenic
primer 11         3' GTC CCA TTG TCT ACG TAG GAC 5'

mutated           5' CAG GGT AAC AGA TGC ATC CTG 3'
coding strand        Gln Gly Asn Arg Cys Ile Leu
```

B: Mutation of Lys 47 to Arg 47

The mutation of Lys 47 to Arg 47 is performed according to example 10 B.

The procedures given above for Examples 3 and 4 are repeated using mutagenic primers 8B and 11 to obtain and characterize the desired mutant protein in which Lys 27 is replaced by Arg 27 and Lys 47 is replaced by Arg 47. The transforming plasmid DNA is referred to as pIN-III-ompA-2/HIR-K27R,K47R. The mutant is designated [$Arg^{27}$,$Arg^{47}$]-desulphatohirudin.

Example 14: Extension of the N-terminus of [$Gln^{27}$,$Gln^{36}$,$Arg^{47}$]-desulphatohirudin with a glycine residue

```
                                 1       3       5
coding strand     signal seq.    Val Val Tyr Thr Asp Cys
of hirudin     5' GCG CAG GCC ... GTT GTT TAC ACC GAC TGC 3'

mutagenic
primer 12      3' CGC GTC CGG CCA CAA CAA ATG TGG CTG ACG 5'
                              ---
mutated        5' GCG CAG GCC GGT GTT GTT TAC ACC GAC TGC 3'
                              ---
coding strand     signal seq. Gly Val Val Tyr Thr Asp Cys
                              ---
```

The procedures given above for Examples 3 and 4 are repeated using mutagenic primers 8A, 8B, 9 and 12 to obtain and characterize the desired mutant protein in which the N-terminus of [$Gln^{27}$,$Gln^{36}$,$Arg^{47}$]-desulphatohirudin is extended by Gly. The protein is designated Glycyl-[$Gln^{27}$,-$Gln^{36}$,$Arg^{47}$]-desulphatohirudin.

Example 15: Extension of the N-terminus of [$Gln^{27}$,$Arg^{47}$]desulphatohirudin with a methionine residue

```
                                 1       3       5
coding strand     signal seq.    Val Val Tyr Thr Asp Cys
of hirudin     5' GCG CAG GCC ... GTT GTT TAC ACC GAC TGC 3'

mutagenic
primer 13      3' CGC GTC CGG TAC CAA CAA ATG TGG CTG ACG 5'
                              ---
mutated        5' GCG CAG GCC ATG GTT GTT TAC ACC GAC TGC 3'
                              ---
coding strand     signal seq. Met Val Val Tyr Thr Asp Cys
                              ---
```

The procedures given above for Examples 3 and 4 are repeated using mutagenic primers 8A, 8B and 13 to obtain and characterize the desired mutant protein in which the N-terminus of [$Gln^{27}$,$Arg^{47}$]-desulphatohirudin is extended by Met. The protein is designated methionyl-[$Gln^{27}$,$Arg^{47}$]-desulphatohirudin.

Example 16: Expression of [$Asn^{27}$]-desulphatohirudin in yeast

[$Asn^{27}$]-desulphatohirudin is a mutant protein with a $Lys^{27} \rightarrow Asn^{27}$ amino acid exchange, which has been achieved by site-directed mutagenesis (see Example 3). Cloning of the coding sequence of the mutated polypeptide in the pIN-III-ompA-2 vector is described in Example 3 II. For expression in yeast the coding sequence with the appropriate mutation is inserted into a suitable yeast expression vector, providing a strong constitutive promoter, a yeast signal sequence and a yeast transcriptional terminator to build a functional expression cassette.

A: Isolation of the coding sequence of [$Asn^{27}$]-desulphatohirudin

25 µg of plasmid pIN-III-ompA-2/HIRK27N (see Example 3 III) are digested with XbaI and BamHI. The 0.3 kb XbaI-BamHI fragment is isolated on a preparative 2 % agarose gel. The DNA is electroeluted and precipitated with ethanol. The XbaI-BamHI fragment comprises the ompA signal sequence and the mutated hirudin coding sequence. The fragment is further cut with HinfI, which cleaves at nucleotide position 22 within the hirudin sequence. The 176 bp HinfI-BamHI fragment is isolated as described above. This DNA fragment codes for [$Asn^{27}$]-disulphatohirudin.

B: Cloning of the coding sequence of [$Asn^{27}$]-desulphatohirudin in a yeast expression vector

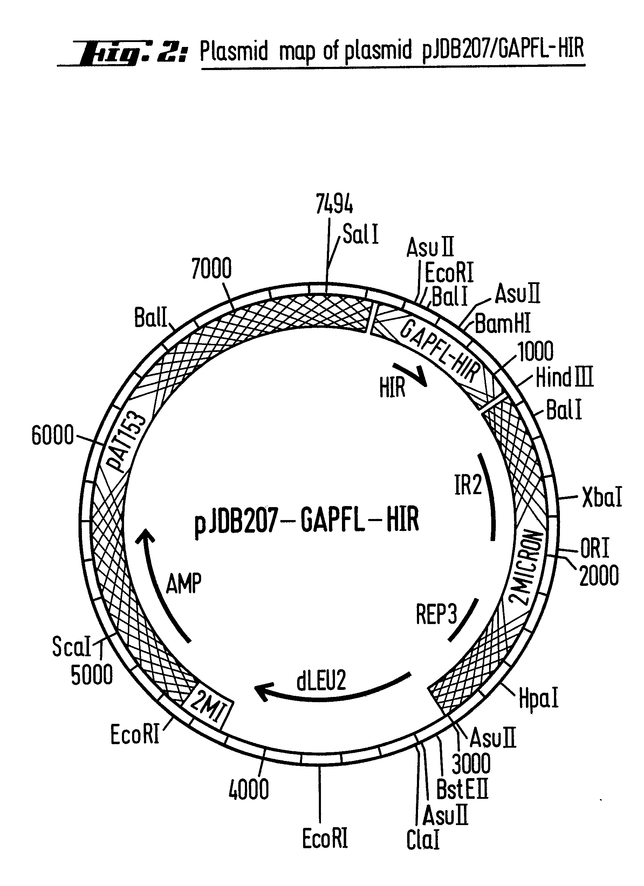

Yeast plasmid pJDB207/GAPFL-HIR (see Fig. 2, European patent application No. 225633) is digested with BalI. There are three BalI sites, one in the PHO5 signal sequence, two in the vector. The 1.3 kb BalI fragment is isolated which contains the pBR322 sequences from the BalI to the BamHI sites, a BamHI/BglII junction to the short, constitutive GAPFL promoter and the PHO5 signal sequence up to the BalI site.

Two synthetic oligonucleotides of the formula

```
            BalI                            HinfI
(1)  5'  CCAATGCAGTTGTTTACACCGACTGCACCG       3'


(2)  3'  GGTTACGTCAACAAATGTGGCTGACGTGGCTTA  5'
```

form a double stranded DNA linker, which provides eight nucleotides from the BalI site to the end of the PHO5 signal sequence and 22 nucleotides of the hirudin coding sequence up to the HinfI site (not indicated in Fig. 2). Oligonucleotides (1) and (2) are each kinased in 10 µl of 60 mM Tris-HCl pH 7.5, 10 mM $MgCl_2$, 5 mM DTT, 0.5 mM ATP and 27 U of T4 polynucleotide kinase (Boehringer) for 45 min at 37°C. The reaction mixtures for oligonucleotides (1) and (2) are combined, heated for 10 min at 75°C and allowed to cool to room temperature. The annealed oligonucleotide linker is stored at -20°C.

Two picomoles of the 1.3 kb BalI DNA fragment is incubated for 16 h at 15°C with a 100 fold excess of the kinased and annealed oligonucleotide linker in 10 µl of 60 mM Tris-HCl pH 7.5, 10 mM $MgCl_2$, 5 mM DTT, 3.5 mM ATP and 400 U of T4 DNA ligase (Biolabs). After inactivation of the T4 DNA ligase for 10 min at 85°C the excess linker is removed by precipitation of the DNA in the presence of 10 mM EDTA, 300 mM sodium acetate pH 6.0 and 0.54 volumes of isopropanol. The DNA is digested with SalI. The resulting fragments are separated on a 1 % preparative agarose gel. The 564 bp fragment is recovered from the gel by electroelution and ethanol precipitation. The DNA is resuspended at a concentration of 0.1 pmoles/µl. The 564 bp SalI-HinfI fragment comprises the 276 bp SalI-BamHI pBR322 DNA fragment, the GAPFL promoter, the PHO5 signal sequence and the coding sequence of hirudin up to nucleotide position 22 (HinfI site).

Plasmid pJDB207/GAPFL-HIR (see above) is digested with SalI and BamHI and the 6.7 kb SalI-BamHI vector fragment is isolated. The vector fragment also contains the PHO5 transcriptional terminator.

Three DNA fragments, isolated as described above, are ligated in the following reaction: 0.2 pmoles of the 564 bp SalI-HinfI fragment, 0.2 pmoles of the 176 bp HinfI-BamHI fragment and 0.1 pmoles of the 6.7 kb SalI-BamHI vector fragment are ligated in 10 µl of 60 mM Tris-HCl pH 7.5, 10 mM $MgCl_2$, 5 mM DTT, 1 mM ATP and 200 U of T4 DNA ligase for 6 h at 15°C. A 1 µl aliquot of the ligation mixture is added to 100 µl of calcium-treated, transformation competent E.coli cells.

12 transformed, ampicillin-resistant colonies are grown in LB medium containing 100 mg/l of ampicillin. Plasmid DNA is prepared [Holmes et. al., Anal. Biochem. 114 (1981), 193] and analysed by BamHI and SalI double digests. The in frame junctions of the linker to the signal sequence and to the hirudin coding sequence are confirmed by DNA sequencing [Sanger et al., Proc. Natl. Acad. Sci. USA 74 (1977), 5463]. One clone with the correct sequence is referred to as pJDB207/GAPFL-HIR-K27N.

In an analogous manner yeast expression plasmids are constructed starting from
pIN-III-ompA-2/HIR-K36N (Example 4),
pIN-III-ompA-2/HIR-K36V (Example 5),
pIN-III-ompA-2/HIR-P48G (Example 6),
pIN-III-ompA-2/HIR-Q49M (Example 7),
pIN-III-ompA-2/HIR-N52M (Example 8),
pIN-III-ompA-2/HIR-H51N (Example 9),
pIN-III-ompA-2/HIR-K27Q,K47R (Example 10),
pIN-III-ompA-2/HIR-K27Q,K36Q,K47R (Example 11),
pIN-III-ompA-2/HIR-K36R,K47R (Example 12) and
pIN-III-ompA-2/HIR-K27R,K47R (Example 13).

The resulting yeast expression plasmids are referred to as
pJDB207/GAPFL-HIR-K36N,
pJDB207/GAPFL-HIR-K36V,
pJDB207/GAPFL-HIR-P48G,
pJDB207/GAPFL-HIR-Q49M,
pJDB207/GAPFL-HIR-N52M,
pJDB207/GAPFL-HIR-H51N,
pJDB207/GAPFL-HIR-K27Q,K47R,
pJDB207/GAPFL-HIR-K27Q,K36Q,K47R,
pJDB207/GAPFL-HIR-K36R,K47R and
pJDB207/GAPFL-HIR-K27R,K47R.

Example 17: Transformation of Saccharomyces cerevisiae strains GRF18 and HT246:

Saccharomyces cerevisiae strains GRF18 (DSM 3665; α, his3-11, his3-15, leu2-3, leu2-112, $can^R$) and HT246 (DSM 4084 a; leu2-3, leu2-112, prb) are transformed with the yeast espression plasmids listed in Example 16B using the transformation protocol described by Hinnen et al. [Proc. Natl. Acad. Sci. USA 75, 1929 (1978)]. Transformed yeast cells are selected on yeast minimal media plates deficient in leucine. Single transformed yeast colonies are isolated and referred to as

Saccharomyces cerevisiae GRF18/pJDB207/GAPFL-HIR-K27N,
Saccharomyces cerevisiae GRF18/pJDB207/GAPFL-HIR-K36N,
Saccharomyces cerevisiae GRF18/pJDB207/GAPFL-HIR-K36V,
Saccharomyces cerevisiae GRF18/pJDB207/GAPFL-HIR-P48G,
Saccharomyces cerevisiae GRF18/pJDB207/GAPFL-HIR-Q49M,
Saccharomyces cerevisiae GRF18/pJDB207/GAPFL-HIR-N52M,
Saccharomyces cerevisiae GRF18/pJDB207/GAPFL-HIR-H51N,
Saccharomyces cerevisiae GRF18/pJDB207/GAPFL-HIR-K27Q,K47R,
Saccharomyces cerevisiae GRF18/pJDB207/GAPFL-HIR-K27Q,K36Q,K47R,
Saccharomyces cerevisiae GRF18/pJDB207/GAPFL-HIR-K36R,K47R
Saccharomyces cerevisiae GRF18/pJDB207/GAPFL-HIR-K27R,K47R,
Saccharomyces cerevisiae HT246/pJDB207/GAPFL-HIR-K27N,
Saccharomyces cerevisiae HT246/pJDB207/GAPFL-HIR-K36N,
Saccharomyces cerevisiae HT246/pJDB207/GAPFL-HIR-K36V,
Saccharomyces cerevisiae HT246/pJDB207/GAPFL-HIR-P48G,
Saccharomyces cerevisiae HT246/pJDB207/GAPFL-HIR-Q49M
Saccharomyces cerevisiae HT246/pJDB207/GAPFL-HIR-N52M,
Saccharomyces cerevisiae HT246/pJDB207/GAPFL-HIR-H51N,
Saccharomyces cerevisiae HT246/pJDB207/GAPFL-HIR-K27Q,K47R,
Saccharomyces cerevisiae HT246/pJDB207/GAPFL-HIR-K27Q,K36Q,K47R,
Saccharomyces cerevisiae HT246/pJDB207/GAPFL-HIR-K36R,K47R and
Saccharomyces cerevisiae HT246/pJDB207/GAPFL-HIR-K27R,K47R.

Example 18: Fermentation of the Saccharomyces cerevisiae transformants

Cells of the S. cerevisiae GRF18 and HT246 transformants are each grown in 10 ml of yeast minimal medium (Difco Yeast Nitrogen Base without aminoacids to which 2 % glucose and 20 mg/l L-histidine are added) in a 50 ml Erlenmeyer flask with shaking at 30°C for 24 hours to a density of 3 x $10^7$ cells/ml.

For the constitutive expression and secretion of the mutated desulphatohirudin species cells are grown in a complex medium consisting of (g/l) peptone (5), yeast extract (10), glucose (20), sucrose (40), ammonium sulphate (3), $KH_2PO_4$ (2), $MgSO_4$ (0.5), NaCl (0.1), $CaCl_2$ (0.1), biotin (10 µg/ml). Approximately $1 \cdot 10^9$ cells/ml are obtained after 48 hours of incubation at 30°C and 200 revs/min.

Example 19: Recovery of desulphatohirudin mutants from S. cerevisiae cultured on a 50 l scale

The culture broth is mixed with Amberlite XAD-7 and is subjected to adsorption for about 4 hours at 25°C. The cells are separated from the resin in a column. After washing with 1M NaCl the resin is eluted with Tris buffer (50 mM, pH 7.0 - 8.5). The main fraction (30 l) is adjusted to pH 2.9 and is applied to a S-Sepharose column (Amicon PA, equilibrated with ammonium formiate buffer 25 mM, pH 2.9) having a bed volume of 2 l. After washing with ammonium formiate buffer (40 mM, pH 3.6) elution is done with ammonium formiate buffer (50 mM, pH 3.8). The main eluate fraction (10 l) is concentrated by means of a Filtron Minisette ultrafiltration system equipped with a Ω 3k membrane. A 0.5 l aliquot of the resulting clear protein solution is applied to a Bio-Gel P-6 fine column (Amicon GF equilibrated with 0.5 % acetic acid) having a bed volume of 1.5 l. Elution is done with 0.5 % acetic acid. The main eluate fraction (1 l) is concentrated by means of ultrafiltration and subsequently applied to a Q-Sepharose fast flow column (Amicon PA, equilibrated with ammonium formiate buffer 25 mM, pH 2.9) having a bed volume of 2 l. Elution is done with ammonium formiate buffer (50 mM, pH 4.2). The main eluate fraction is concentrated by means of ultrafiltration and is subsequently diafiltrated against water. The resulting clear aqueous solution is lyophilised. The solid consists of the pure desulphatohirudin mutant.

Example 20: Kinetic characterization

A. Preparation and characterization of thrombin:

Thrombin is purified and characterized as described by S.R. Stone and J. Hofsteenge [Biochemistry 25, 4622-4628 (1986)]. The concentration of active thrombin is determined by active site titration with 4-methylumbelliferyl p-guanidinobenzoate [Jameson, G.W., Roberts, D.V., Adams, R.W., Kyle, W.S.A. and Elmore, D.T., Biochem. J. 131, 101-117 (1973)].

B. Enzyme assays:

The release of p-nitroaniline that results from the hydrolysis of the peptidyl p-nitroanilide substrates by thrombin is followed by measuring the increase in absorbance at 405 nm with a Shimadzu UV 240 spectrophotometer. The assays are performed in polystyrene cuvettes at 37°C in 0.05 M Tris-HCl buffer, pH 7.8, which contains 0.1 % polyethyleneglycol 6000, 0.1 M NaCl and p-nitroanilide substrate. The substrate D-Val-Leu-Arg-p-nitroanilide (S-2266; Kabi Vitrum) is used at a concentration of 300 µM in tight-binding titration experiments which are used to determine the concentration of the mutant hirudins. For such assays a thrombin concentration of 1.0 nM is used. The substrate D-Phe-pipecolyl-Arg-p-nitroanilide (S-2238; Kabi Vitrum) at a concentration of 100 µM and a thrombin concentration of 20 to 50 pM are used in slowing-binding inhibition experiments to determine the kinetic characteristics of the mutant hirudins (see below). Assays are started by the addition of thrombin. The amount of product formed is calculated using an absorption coefficient of 9,920 $M^{-1}cm^{-1}$ for p-nitroaniline at 405 nm and the concentration of the substrate is determined spectrophotometrically at 342 nm using an absorption coefficient of 8,270 $M^{-1}cm^{-1}$ [Lottenberg, R. & Jackson C.M., Biochim. Biophys. Acta 742, 558-564 (1983)].

C. Kinetic theory and data analysis

Tight-binding titration:

The inhibition of an enzyme in the presence of a substrate can be represented by the following mechanism (Scheme 1):

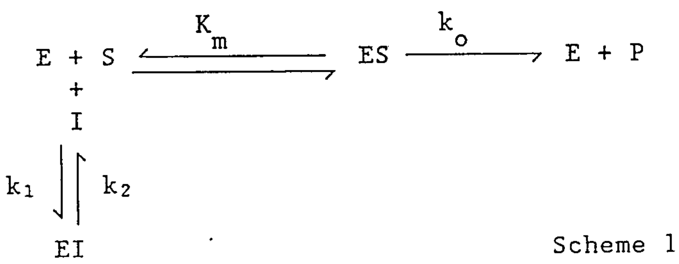


Scheme 1

The dissociation of the inhibitor ($K_I$) is equal to $k_2/k_1$. If the binding of the inhibitor to the enzyme causes a significant depletion in the concentration of the free inhibitor, the variation of the steady-state velocity ($v_s$) with the total inhibitor concentration ($I_t$) will be described by equation (1) [Morrison, J.F., Biochim. Biophys. Acta 185, 269-286 (1969); Henderson, P.J.F., Biochem. J. 127, 321-333 (1972)]:

$$v_s = \frac{v_o}{2E_t} [sqr((K_{I'} + I_t - E_t)^2 + 4K_{I'}E_t) - (K_{I'} + I_t - E_t)] \quad (1)$$

where $v_o$ is the velocity observed in the absence of the inhibitor, $E_t$ is the total enzyme concentration, and $K_{I'}$ is the apparent inhibition constant. If the inhibitor and the substrate compete for the active site, $K_{I'}$ is related to the dissociation constant of the inhibitor ($K_I$) by equation (2):

$K_{I'} = K_I(1 - S/K_m)$ (2)

where S is the concentration of substrate and $K_m$ is its Michaelis constant. If the concentration of the enzyme or inhibitor is not accurately known, an additional factor can be incorporated into equation (1) to allow for this fact [Williams, J.W., and Morrison, J.F., Methods Enzymol. 63, 437-467 (1979)]. For the inhibition of thrombin by hirudin, the concentration of thrombin is known from active-site titration, but the concentration of hirudin is only known by weight and the data can be analysed according to equation (3):

$$v_s = \frac{v_o}{2E_t} [sqr((K_{I'} + xI_x - E_t)^2 + 4K_{I'}E_t) - (K_{I'} + xI_x - E_t)] \quad (3)$$

where $I_x$ is the concentration of the inhibitor in terms of weight per volume and x is a factor which, when multiplied together with $I_x$, will yield the molar concentration of the inhibitor.

In tight-binding titration experiment, the dissociation constant for hirudin and its concentration is determined by holding the concentration of thrombin constant and varying the concentration of hirudin over a range which includes several concentrations that are lower than the concentration of thrombin and several concentrations that are higher. The total number of data points is usually between 7 and 10. The steady-state velocities obtained at each hirudin concentration is then fitted by weighted nonlinear regression to equation (3). For the regression, the observed values for the steady-state velocity are weighted according to the reciprocal of their value. This type of weighting has been empirically found to give the best results [Stone, S.R, and Hofsteenge, J., Biochemistry, 25, 4622-4628 (1986)].

Slow, tight-binding inhibition:

If the rate of interaction of the inhibitor with the enzyme is slow so that the inhibited steady-state velocity is only slowly achieved, the progress curve of product formation for the mechanism of Scheme 1 will be described by equation (4) [Cha, S., Biochem. Pharmacol. 25, 2695-2702 (1976); Williams, J.W., Morrison, J.F., and Duggleby, R.G., Biochemistry 18, 2567-2573 (1979)]:

$$P = v_s . t + \frac{(v_o - v_s)(1 - d)}{d.k'} \log \left(\frac{1 - d.\exp(-k'.t)}{1 - d}\right) \quad (4)$$

where P is the amount of product formed at time t, d is a function of $E_t$, $I_t$ and $K_{I'}$, and k′ is a function of these parameters and the observed second-order association rate constant ($k_{1'}$) for the interaction between the inhibitor and enzyme (Cha et al., supra). In order to determine the kinetic parameters for the mutant hirudins, progress-curve data obtained at at least six different hirudin concentrations are fitted to equation (4) by nonlinear regression. This analysis yields estimates for $K_{I'}$, $k_{1'}$ and the observed dissociation rate constant ($k_{2'}$). It has previously been shown that the value of $k_{1'}$ is independent of the binding of the substrate at the active site but that the observed values of $k_{2'}$ and $K_{I'}$ should be divided by (1 + [S]/$K_m$) in order to obtain the true values ($k_2$ and $K_I$; S.R. Stone and J. Hofsteenge, Biochemistry 25, 4622-4628 (1986); Stone, S.R., Braun, P.J., and Hofsteenge, J., Biochemistry 26, 4617-4624 (1987)]. For these calculations, the previously determined value of 3.6 μM for the $K_m$ of D-Phe-pipecolyl-Arg-p-nitroanilide is used [Hofsteenge, J., Taguchi, H., and Stone, S.R., Biochem. J. 237, 243-251 (1986)]. Using these kinetic methods the following values for $K_I$ and $k_1$ are obtained:

| form of desulphato-hirudin (=H) | $10^{-8} \cdot k_1$ $M^{-1}.s^{-1}$ | $K_I$ (pM) |
|---|---|---|
| recombinant desulphatohirudin | 1.37 ± 0.03 | 0.231 ± 0.006 |
| [$Gln^{27}$, $Arg^{47}$]-H | 1.08 ± 0.08 | 1.85 ± 0.04 |
| [$Gln^{27}$, $Gln^{36}$, $Arg^{47}$]-H | n.d. | 1.39 ± 0.02 |

(n.d. = not determined)

Example 21: Pharmaceutical composition containing a desulphatohirudin mutant for parenteral administration

A solution containing a desulphatohirudin mutant according to any one of the preceding Examples is dialysed against a 0.9 % NaCl solution. The concentration of the solution is then adjusted by diluting with the same NaCl solution to 0.2 mg/ml or 2 mg/ml. These solutions are sterilized by ultrafiltration (membranes with 0.22 μm pores).

The sterilized solutions can be used directly, for example for intravenous administration.

Deposit of microorganisms

The following microorganisms were deposited at the Deutsche Sammlung von Mikroorganismen (DSM), Mascheroder Weg 1b, D-3300 Braunschweig (FRG).

| microorganismus | deposition date | accession number |
|---|---|---|
| Saccharomyces cerevisiae GRF18 | March 4, 1986 | DSM 3665 |
| Saccharomyces cerevisiae HT246 | April 15, 1987 | DSM 4084 |

**Claims**

1. Mutants of desulphatohirudin having the amino acid sequence

$$
\begin{array}{l}
\text{H-Z}_0\text{-Val-Tyr-Thr-Asp-Cys-Thr-Glu-Ser-Gly-} \quad ^{10}\\
\text{-Gln-Asn-Leu-Cys-Leu-Cys-Glu-Gly-Ser-Asn-} \quad ^{20}\\
\text{-Val-Cys-Gly-Gln-Gly-Asn-Z}_1\text{-Cys-Ile-Leu-} \quad ^{30}\\
\text{-Gly-Ser-Asp-Gly-Glu-Z}_2\text{-Asn-Gln-Cys-Val-} \quad ^{40}\\
\text{-Thr-Gly-Glu-Gly-Thr-Pro-Z}_3\text{-Z}_4\text{-Z}_5\text{-Ser-} \quad ^{50}\\
\text{-Z}_6\text{-Z}_7\text{-Asp-Gly-Asp-Phe-Glu-Glu-Ile-Pro-} \quad ^{60}\\
\text{-Glu-Glu-Tyr-Leu-Gln-OH}
\end{array}
$$

(I)

wherein $Z_o$ represents Val or the dipeptidyl radicals Gly-Val or Met-Val, $Z_1$ is Lys, Gln, Asn, Leu, Arg or Val, $Z_2$ represents Lys, Arg, Asn, Val, Leu or Gln, $Z_3$ represents Lys, Arg, Asn, Val or Leu, $Z_4$ represents Pro or Gly, $Z_5$ and $Z_7$ independently from each other represent Gln, Asn or Met, and $Z_6$ represents His, Gln or Asn, with the exception of compounds of the formula I in which $Z_o$ represents Val, $Z_1$ represents Lys or Gln, $Z_2$ represents Lys or Gln, $Z_3$ represents Lys, $Z_4$ represents Pro, $Z_5$ represents Gln, $Z_6$ represents Gln or His and $Z_7$ represents Asn, and salts thereof.

2. Desulphatohirudin mutants of the formula I according to claim 1 wherein $Z_o$ represents Val or the dipeptidyl radicals Gly-Val or Met-Val, $Z_1$ represents Lys, Gln, Asn or Arg, $Z_2$ represents Lys, Arg, Asn, Val or Gln, $Z_3$ represents Lys or Arg, $Z_4$ represents Pro or Gly, $Z_5$ represents Gln or Met, $Z_6$ represents His or Asn and $Z_7$ represents Asn or Met, with the exception of compounds of the formula I in which $Z_o$ represents Val, $Z_1$ represents Lys or Gln, $Z_2$ represents Lys or Gln, $Z_3$ represents Lys, $Z_4$ represents Pro, $Z_5$ represents Gln, $Z_6$ represents His and $Z_7$ represents Asn, and salts thereof.

3. [$Asn^{27}$]-desulphatohirudin according to claim 1.

4. [$Asn^{36}$]-desulphatohirudin according to claim 1.

5. [$Val^{36}$]-desulphatohirudin according to claim 1.

6. [$Gly^{48}$]-desulphatohirudin according to claim 1.

7. [$Met^{49}$]-desulphatohirudin according to claim 1.

8. [$Met^{52}$]-desulphatohirudin according to claim 1.

9. [$Asn^{51}$]-desulphatohirudin according to claim 1.

10. [$Gln^{27}$, $Arg^{47}$]-desulphatohirudin according to claim 1.

11. [$Gln^{27}$, $Gln^{36}$, $Arg^{47}$]-desulphatohirudin according to claim 1.

12. [$Arg^{36}$, $Arg^{47}$]-desulphatohirudin according to claim 1.

13. [$Arg^{27}$, $Arg^{47}$]-desulphatohirudin according to claim 1.

14. Glycyl-[$Gln^{27}$, $Gln^{36}$, $Arg^{47}$]-desulphatohirudin according to claim 1.

15. Methionyl-[$Gln^{27}$,$Arg^{47}$]-desulphatohirudin according to claim 1.

16. A pharmaceutical composition comprising a desulphatohirudin mutant according to claim 1.

17. A desulphatohirudin mutant according to claim 1 for use in a method for the prophylactic or therapeutic treatment of the human or animal body.

18. Method for the production of a desulphatohirudin mutant according to claim 1 comprising culturing a microbial host strain which has been transformed with a hybrid vector comprising an expression cassette consisting of a promoter operably linked to a first DNA sequence encoding a signal peptide linked in the proper reading frame to a second DNA sequence coding for the desulphatohirudin mutant, and a DNA sequence containing transcription termination signals, and isolating the desulphatohirudin mutant, and, if desired, converting an obtained polypeptide having free carboxy and/or amino groups into a salt or converting a salt obtained into the free compound.

19. A desulphatohirudin mutant obtainable by the method according to claim 18.

20. A hybrid vector comprising an expression cassette consisting of a promoter operably linked to a first DNA sequence encoding a signal peptide linked in the proper reading frame to a second DNA sequence coding for a desulphatohirudin mutant according to claim 1, and a DNA sequence containing transcription termination signals.

21. Method for the production of a hybrid vector according to claim 20 comprising linking the expression cassette consisting of a promoter operably linked to a first DNA sequence encoding a signal peptide linked in the proper reading frame to a second DNA sequence coding for the desulphatohirudin mutant, and a DNA sequence containing transcription termination signals, or the constituents of the expression cassette to the DNA fragments containing selective genetic markers and origins of replication for the selection microbial host in the predetermined order.

22. A microbial host strain which has been transformed with a hybrid vector comprising an expression cassette consisting of a promoter operably linked to a first DNA sequence encoding a signal peptide linked in the proper reading frame to a second DNA sequence coding for a desulphatohirudin mutant according to claim 1, and a DNA sequence containing transcription termination signals.

23. Method for the production of the transformed microbial host strain according to claim 22 comprising transforming a microbial host strain with the hybrid vector according to claim 20.

**Claims for the following contracting states: GR, ES**

1. A process for the production of mutants of desulphatohirudin having the amino acid sequence

```
                                      10
H-Z -Val-Tyr-Thr-Asp-Cys-Thr-Glu-Ser-Gly-
   o
                                       20
 -Gln-Asn-Leu-Cys-Leu-Cys-Glu-Gly-Ser-Asn-
                                      30
 -Val-Cys-Gly-Gln-Gly-Asn-Z1-Cys-Ile-Leu-
                                      40
 -Gly-Ser-Asp-Gly-Glu-Z2-Asn-Gln-Cys-Val-
                                    50
 -Thr-Gly-Glu-Gly-Thr-Pro-Z3-Z4-Z5-Ser-
                                     60
 -Z6-Z7-Asp-Gly-Asp-Phe-Glu-Glu-Ile-Pro-

 -Glu-Glu-Tyr-Leu-Gln-OH
```

(I)

wherein $Z_o$ represents Val or the dipeptidyl radicals Gly-Val or Met-Val, $Z_1$ is Lys, Gln, Asn, Leu, Arg or Val, $Z_2$ represents Lys, Arg, Asn, Val, Leu or Gln, $Z_3$ represents Lys, Arg, Asn, Val or Leu, $Z_4$ represents Pro or Gly, $Z_5$ and $Z_7$ independently from each other represent Gln, Asn or Met, and $Z_6$ represents His, Gln or Asn, with the exception of compounds of the formula I in which $Z_o$ represents Val, $Z_1$ represents Lys or Gln, $Z_2$ represents Lys or Gln, $Z_3$ represents Lys, $Z_4$ represents Pro, $Z_5$ represents Gln, $Z_6$ represents Gln or His and $Z_7$ represents Asn, and salts thereof, which process comprises culturing a microbial host strain which has been transformed with a hybrid vector comprising an expression cassette consisting of a promoter operably linked to a first DNA sequence encoding a signal peptide linked in the proper reading frame to a second DNA sequence coding for the desulphatohirudin mutant, and a DNA sequence containing transcription termination signals, and isolating the desul phatohirudin mutant, and, if desired, converting an obtained polypeptide having free carboxy and/or amino groups into a salt or converting a salt obtained into the free compound.

2. A process for the production of desulphatohirudin mutants of the formula I according to claim 1 wherein $Z_o$ represents Val or the dipeptidyl radicals Gly-Val or Met-Val, $Z_1$ represents Lys, Gln, Asn or Arg, $Z_2$ represents Lys, Arg, Asn, Val or Gln, $Z_3$ represents Lys or Arg, $Z_4$ represents Pro or Gly, $Z_5$ represents Gln or Met, $Z_6$ represents His or Asn and $Z_7$ represents Asn or Met, with the exception of compounds of the formula I in which $Z_o$ represents Val, $Z_1$ represents Lys or Gln, $Z_2$ represents Lys or Gln, $Z_3$ represents Lys, $Z_4$ represents Pro, $Z_5$ represents Gln, $Z_6$ represents His and $Z_7$ represents Asn, and salts thereof.

3. A process for the production of [$Asn^{27}$]-desulphatohirudin according to claim 1.

4. A process for the production of [$Asn^{36}$]-desulphatohirudin according to claim 1.

5. A process for the production of [$Val^{36}$]-desulphatohirudin according to claim 1.

6. A process for the production of [$Gly^{48}$]-desulphatohirudin according to claim 1.
7. A process for the production of [$Met^{49}$]-desulphatohirudin according to claim 1.
8. A process for the production of [$Met^{52}$]-desulphatohirudin according to claim 1.
9. A process for the production of [$Asn^{51}$]-desulphatohirudin according to claim 1.
10. A process for the production of [$Gln^{27}$, $Arg^{47}$]-desulphatohirudin according to claim 1.
11. A process for the production of [$Gln^{27}$, $Gln^{36}$, $Arg^{47}$]-desulphatohirudin according to claim 1.
12. A process for the production of [$Arg^{36}$, $Arg^{47}$]-desulphatohirudin according to claim 1.
13. A process for the production of [$Arg^{27}$, $Arg^{47}$]-desulphatohirudin according to claim 1.
14. A process for the production of Glycyl- [$Gln^{27}$, $Gln^{36}$, $Arg^{47}$]-desulphatohirudin according to claim 1.
15. A process for the production of Methionyl-[$Gln^{27}$, $Arg^{47}$]-desulphatohirudin according to claim 1.
16. A process for the production of a pharmaceutical composition which process comprises admixing a desulphatohirudin mutant of the formula I that can be produced in accordance with claim 1, or a pharmaceutically acceptable salt thereof, to a pharmaceutically acceptable carrier and/or adjuncts.
17. A process for the production of a hybrid vector comprising an expression cassette consisting of a promoter operably linked to a first DNA sequence encoding a signal peptide linked in the proper reading frame to a second DNA sequence coding for a desulphatohirudin mutant that can be produced according to claim 1, and a DNA sequence containing transcription termination signals, which process comprises linking the expression cassette consisting of a promoter operably linked to a first DNA sequence encoding a signal peptide linked in the proper reading frame to a second DNA sequence coding for the desulphatohirudin mutant, and a DNA sequence containing transcription termination signals, or the constituents of the expression cassette to the DNA fragments containing selective genetic markers and origins of replication for the selected microbial host in the predetermined order.
18. A process for the production of a microbial host strain which has been transformed with a hybrid vector comprising an expression cassette consisting of a promoter operably linked to a first DNA sequence encoding a signal peptide linked in the proper reading frame to a second DNA sequence coding for a desulphatohirudin mutant that can be produced according to claim 1, and a DNA sequence containing transcription termination signals, which process comprises transforming a microbial host strain with said hybrid vector.
19. A microbial host strain obtainable by the process according to claim 18.

Fig. 1: Construction of plasmid pML350

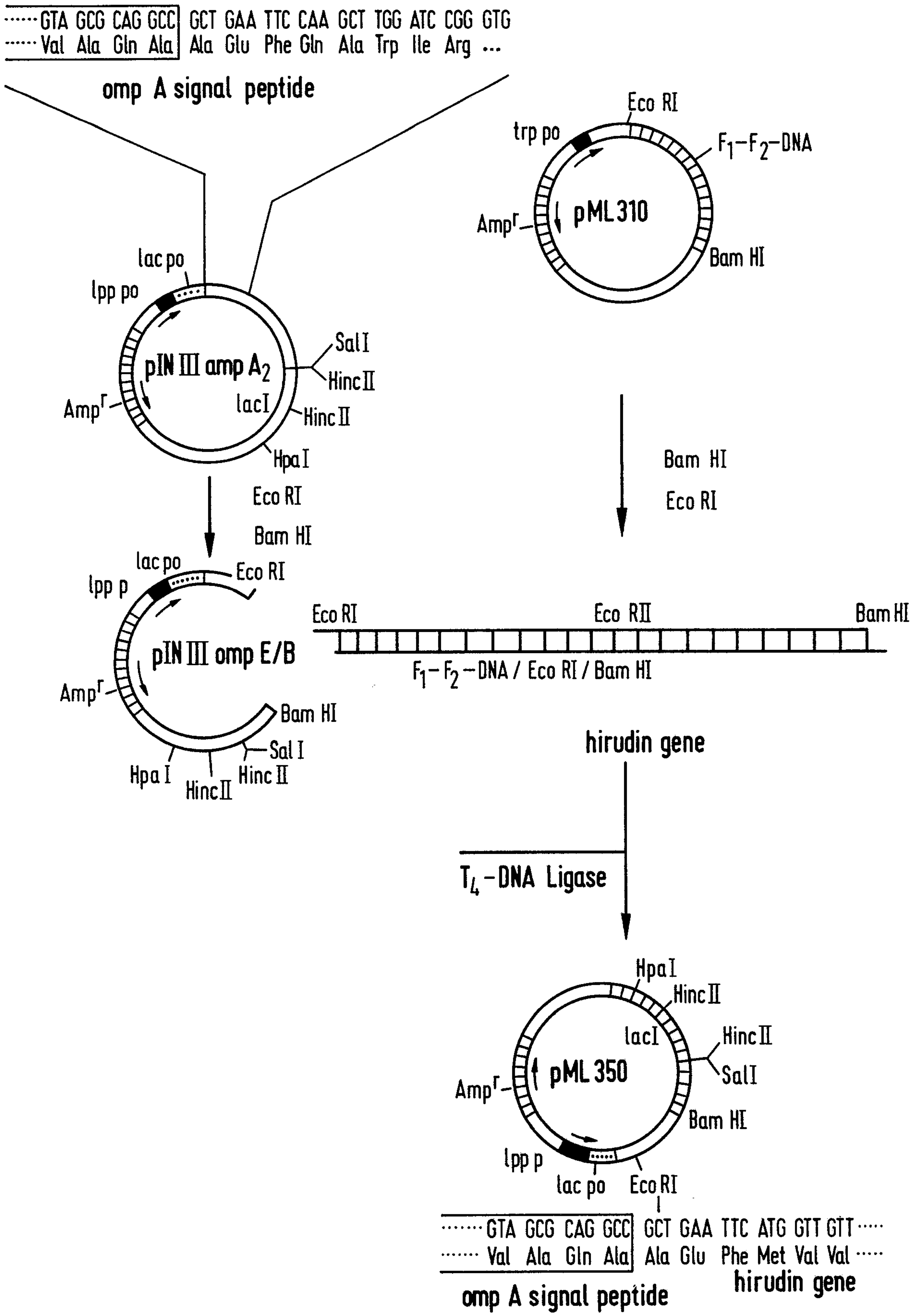

Fig. 2: Plasmid map of plasmid pJDB207/GAPFL-HIR